(19) Europäisches Patentamt / European Patent Office / Office européen des brevets

(11) EP 2 969 021 B1

(12) EUROPEAN PATENT SPECIFICATION

(45) Date of publication and mention of the grant of the patent:
07.08.2019 Bulletin 2019/32

(51) Int Cl.:
A61Q 5/06 (2006.01)     A61Q 5/02 (2006.01)
A61Q 5/12 (2006.01)     A61K 8/81 (2006.01)
A61K 8/02 (2006.01)     A61K 8/04 (2006.01)

(21) Application number: 14764854.7

(22) Date of filing: 14.03.2014

(86) International application number:
PCT/US2014/028334

(87) International publication number:
WO 2014/144076 (18.09.2014 Gazette 2014/38)

(54) HAIR CARE COMPOSITIONS COMPRISING POLYELECTROLYTE COMPLEXES FOR DURABLE BENEFITS

HAARPFLEGEZUSAMMENSETZUNGEN MIT POLYELEKTROLYTKOMPLEXEN FÜR DAUERHAFTE VORTEILE

COMPOSITIONS DE SOINS CAPILLAIRES COMPRENANT DES COMPLEXES DE POLYÉLECTROLYTES POUR DES BIENFAITS DE LONGUE DURÉE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 15.03.2013 US 201361792035 P

(43) Date of publication of application:
20.01.2016 Bulletin 2016/03

(73) Proprietor: ISP Investments LLC
Wilmington, DE 19805 (US)

(72) Inventors:
• ZHOU, Yan
  Branchburg, New Jersey 07045 (US)
• RIGOLETTO, Raymond Jr.
  Denville, New Jersey 07834 (US)
• FOLTIS, Linda C.
  Nutley, New Jersey 07110 (US)
• QU, Xin
  Shanghai (CN)
• COLACO, Allwyn
  Morristown, New Jersey 07960 (US)

(74) Representative: Kutzenberger Wolff & Partner
Waidmarkt 11
50676 Köln (DE)

(56) References cited:
WO-A1-2005/004821      WO-A1-2009/098638
WO-A1-2011/126978      WO-A1-2011/126978
WO-A1-2012/075274      WO-A2-01/85819
WO-A2-2004/096895      WO-A2-2012/054243
WO-A2-2012/054243      WO-A2-2014/020081
US-A- 4 240 450         US-A- 4 299 817
US-A1- 2006 251 603     US-A1- 2011 180 092
US-A1- 2011 256 085

Remarks:
The file contains technical information submitted after the application was filed and not included in this specification

EP 2 969 021 B1

**Description**

**FIELD OF THE INVENTION**

The present application relates to hair care compositions, and, more particularly, to hair care compositions containing polyelectrolyte complexes for durable benefits.

**BACKGROUND OF THE INVENTION**

Current trend of hair style fashion indicates that consumers have increasing desire to temporally change their hair appearance so as to keep their hair style to the latest hair style fashion or express their individuality and personal style to a greater extent. The methods to achieve temporary or non-permanent shaping or style of hair include using styling products such as hair spray, mousse, styling gel in combination with styling implements such as flat irons to make straight hair style and curling iron and hair rollers to form curls on hair, which allows one to have a different hair style even from one day to the next. The temporary setting of hair created by these traditional methods lasts not long, typically a few hours or up to a day and it disappears when hair becomes wet under high humidity condition or is washed. Therefore, a durable non permanent styling that can last a few days and survive multiple shampoo washes is highly desired as many consumers want to retain a nice temporary hair style longer beyond multiple wash cycles, especially maintain good curl retention under high humidity condition after hair is washed, reducing the frequency of repeatedly treating the hair with a styling product. Therefore, there is a need for a composition and methods which can offer benefit of durable non-permanent hair styling and/or wash-resistant retention of shape of hair fibers over multiple shampoo washes and multiple conditioner rinsing.

Accordingly, it is an objective of the present application to provide compositions and methods which offer the benefit of durable non-permanent hair styling and/or wash-resistant retention of shape of hair fibers over multiple shampoo washes and multiple conditioner rinsing. Such a durable styling composition will offer significant advantage of use convenience to consumers along with its economical benefit because there will be reduced frequency to re-apply the styling compositions to hair while the desired style of hair is still maintained.

The WO Publication Number 2011 1269778A1 of ISP Investments Inc. discloses a sprayable composition comprising: a complex of (A) at least one charged (or pseudo-charged) polymer having a molecular weight of about 125,000 amu or more; and (B) a least one oppositely charged, rheology modifying, crosslinked polymer having at least one carboxylic functional group. The composition may provide a spray with a median particle size of less than about 175 [mu]. Also disclosed are methods for preparing the compositions as well as creating the spray. The invention enables the spray of the high molecular weight charged (or pseudo-charged) polymer in any number of uses including personal care and performance chemicals application.

The United State Patent number 7,837,983B2 of ISP Investments Inc. describes a hair care composition for mending split ends comprising a polyelectrolyte complex between (a) a cationic polyquaternium polymer and (b) an anionic copolymer containing mono-, di- or tri-acid groups, or salts thereof, in a charge ratio of (a):(b) of 0.82 to 1.80; wherein said polyelectrolyte complex has a microgel structure.

The US Publication Number 20120213723 of L'Oreal discloses a heat-activated composition for durable non-permanent shaping or durable retention of a non-permanent shape of least one keratinous fiber comprising: (a) at least one film forming agent: and (b) at least one saccharide type compound chosen from $C_3$ to $C_5$ monosaccharides, optionally substituted with at least one $_{C1}$ to $_{C22}$ carbon chain, and compounds comprising at least one $C_5$ to $C_7$ saccharide unit substituted with at least one amino group.

The WO Publication Number 2012075274A1 of ISP Investments Inc. discloses a heat-activated hair styling composition comprising at least: (A) a compound having at least one of diacid, diester, half-acid/half-ester, or anhydride functionality, and (B) a compound having at least one amine moiety or hydroxyl group, wherein said (A) is the same as or different from said (B), wherein said (A) is a polymer, an oligomer, a small molecule. More specifically, it has described a hair care composition comprising (i) diacid and (ii) polyamide-1 for heat-activated durable styling benefit for hair through succinimide linkage formation between the two polymers and hair fibers amino acid group once heat treated.

The US Publication Number 20060188468A1 of BASF discloses a cosmetic or pharmaceutical composition comprising A) at least one water soluble or water dispersible polyelectrolyte complex comprising A1) at least one polymer with cationogenic groups which comprises, in copolymerized form, vinylimidazole and/or a derivative thereof and at least one further monomer copolymerizable therewith, and A2) at least one acid-group-containing polymer, and B) at least one cosmetically acceptable carrier, wherein said composition is used in hair-treatment compositions as setting agents and/or as conditioners in the form of a hair gel, shampoo, setting foam, hair tonic, hairspray or hair mousse.

## SUMMARY OF THE INVENTION

The primary objective of the present application is to provide a hair care composition which is capable of providing durable non-permanent hair styling and/or wash-resistant retention of shape of hair fibers, and wherein, such durable styling can survive over a number of shampoo wash cycles and/ or multiple conditioner rinsing.

Accordingly, it is one aspect of the present application to provide an aqueous hair care composition which is capable of providing durable non-permanent hair styling and/or wash-resistant retention of shape of hair fibers comprising a polyelectrolyte complex **according to claim 1.**

Another aspect of the present application is to provide a method for providing durable non-permanent hair styling and/or wash resistant retention of shape of hair fibers comprising the steps of: (i) applying said durable styling hair composition to the hair fibers; (ii) drying the hair fibers with a hair drier and/or style the dried hair to desired shape: and (iii) optionally, heating the hair fibers to a temperature ranging from about 90°C to about 240°C using a hair healing device such as hot flat iron, curling iron and/or a hair drier; and (iv) after shampooing the hair next day or two, drying and styling the hair to desired shape without further need to apply the PEC composition until re-application is needed after multiple shampoo wash and conditioner rinse cycles, typically after 3 to 5 wash cycles or more.

One important aspect of the present application is to provide a process for preparing an aqueous hair care composition capable of providing durable non-permanent hair styling and/or wash-resistant retention of shape of hair libers comprising the steps of: (i) preparing an aqueous solution of about 0.1 wt. % to about 20 wt. % of at least one cationic polymer (B): (ii) adding about 0.1 wt. % to about 3 wt. % of thickeners to the solution of step (i) and mixing continuously until to form an uniform mixture: (iii) mixing the oil phase ingredients of (a) about 0.1 wt. % to about 10 wt. % of emulsifier. (b) about 0.1 wt. % to 10 wt. % of cationic surfactants and (c) optionally, about 0.5 wt. % to about 5 wt. % of silicone in a different container and heating the mixture to 70°C to 75°C to form a uniform mixture of oil phase and adding the resultant mixture oil phase to the uniform mixture of step (ii) which is also heated to the same temperature with continuous mixing for about 20 to about 30 minutes, and allowing the resultant to cool to 35°C and slow down mixing: (iv) neutralizing about 0.01% wt. % to about 20 wt. % of at least one anionic polymer (A) employing an aqueous alkali medium to bring the pH of about 7.0 and adding this resultant solution to the resultant of step (iii) at 35°C with continuous mixing until to form an uniform mixture; and (v) adding at least one about 0.1 wt. % to 5 wt. % of other cosmetic additives such as preservative and perfume to the resultant of step (iv) and pass through homogenizer if required.

In another aspect of the present application there is provided a method of providing durable non-permanent hair styling of hair fibers is capable of exhibiting styleability, volume, shine, hair manageability, durability of style, softness, case of styling, anti-frizz, hair fiber alignment, greater wet conditioning, dry conditioning, wet combing, case of use with styling appliances, or hair protection from styling appliances in comparison with a method which comprises a hair composition that does not comprise said polyelectrolyte complex.

One important aspect of the present application is to provide an economical benefit and use convenience for consumers by enabling them with a hair styling composition which can provide durable non-permanent hair styling and/or aqueous wash-resistant retention of shape of hair fibers, and wherein the durable styling benefit can last for at least 2 shampoo wash cycles and/or conditioner rinse cycles of up to 5 or more.

## BRIEF DESCRIPTION OF THE FIGURES

Further embodiments of the present invention can be understood with the appended figures.

**FIG. 1** is an illustration of Example 1 comprising 0.45% Methylvinyl ether/maleic anhydride coplolymer and 4.3% Polyquaternium-55.

**FIG. 2** is an illustration of Example 2 comprising 0.728% Methylvinyl ether/ maleic anhydride coplolymer and 3.26% of Polyquatemium-55.

**FIG. 3** is an illustration of comparison of Example 3 comprising (i) 0.45% Methylvinyl ether/ maleic anhydride coplolymer; and (ii) 1.96% of Polyquatemium-55.

**FIG. 4** shows Hair Salon test of durable styling cream comprising PEC in Example 14.

**FIG. 5** shows Hair Salon test of durable styling cream comprising PEC in Example 14.

**FIG. 6** shows Hair tress test of hair durable styling lotion comprising PEC in Example 15 for hair styling benefit.

**FIG. 7** shows Hair Salon test of styling conditioner rinse comprising PEC in Example 16.

## DETAILE DESCRIPTION OF THE INVENTION

While this specification concludes with claims particularly pointing out and distinctly claiming that which is regarded as the invention, it is anticipated that the invention can he more readily understood through reading the following detailed description of the invention and study of the included examples.

The singular forms "a." "an." and "the" include plural forms unless the context clearly dictates otherwise specified or clearly implied to the contrary by the context in which the reference is made. The term "Comprising" and "Comprises of" includes the more restrictive claims such as "Consisting essentially of" and "Consisting of.

The term "about" can indicate a difference of 10 percent of the value specified. Numerical ranges as used herein are meant to include every number and subset of numbers enclosed within that range, whether particularly disclosed or not. Further, these numerical ranges should be construed as providing support for a claim directed to any number or subset of numbers in that range.

All percentages, parts, proportions and ratios as used herein, are by weight of the total composition, unless otherwise specified. All such weights as they pertain to listed ingredients are based on the active level and, therefore; do not include solvents or byproducts that may be included in commercially available materials, unless otherwise specified.

All references to singular characteristics or limitations of the present invention shall include the corresponding plural characteristic or limitation, and *vice-versa,* unless otherwise specified or clearly implied to the contrary by the context in which the reference is made.

As used herein, the words "preferred" or "preferably" and variants refer to embodiments of the invention that afford certain benefits, under certain circumstances. However, other embodiments may also he preferred, under the same or other circumstances. Furthermore, the recitation of one or more preferred embodiments does not imply that other embodiments arc not useful, and is not intended to exclude other embodiments from the scope of the invention.

References herein to "one embodiment" or "one aspect" or "one version" or "one objective" of the invention include one or more such embodiment, aspect, version or objective, unless the context clearly dictates otherwise.

All publications, articles, papers, patents, patent publications, and other references cited herein are hereby incorporated herein in their entirety for all purposes to the extent consistent with the disclosure herein.

The term "at least one" as used herein means one or more and thus includes individual components as well as mixtures/combinations.

The term "monomer" refers to the repeat units that comprise a polymer. A monomer is a compound that chemically bonds to other molecules, including other monomers, to form a polymer.

The term "polymer" refers to both linear and branched polymers derived from one or more monomer units, which may or may not be crosslinked, or grafted. Non-limiting examples of polymers include copolymers, terpolymers, tetramers, and the like, wherein the polymer is random, block, or alternating polymer.

The term "non-homopolymer" refers to a polymer that comprises more than one type of monomer, and includes such polymers wherein a small amount of polymerization solvent may be covalently bonded into the polymer. The non-homopolymer is copolymers, terpolymers, tetramers, and the like.

The term "cationic polymers" refers to polymers displaying at least one primary amine, secondary amine or tertiary amine or quaternary ammonium group in their principal chain or branched chain or in the substituted form.

The term "Charge density" as used herein refers to the ratio of the number of charges on a polymer unit to the molecular weight of said polymer unit. Charge Density is further defined as the number of miliequivalents of charge per gram (*meq/g*) of polymer.

The term "Charge ratio" as used herein refers to ratio of the cationic species to the anionic species, and specifically a charge ratio refers to a quantitative relationship between net positive charges contributed by cationic or pseudocationic polymer (B) and net negative charges contributed by the anionic polymer (A) of the polyelectrolyte complex of the present application.

$$\text{Charge Ratio} = \frac{\text{Total number of moles of positive (cationic) charges}}{\text{Total number of moles of negative (anionic) charges}}$$

According to one embodiment of the present application, the polyelectrolyte complex (PEC) comprises formation of macro-ion complex between at least two polymers which are oppositely charged polyelectrolytes, and wherein, the formation of complex is mainly driven by electrostatic attraction between the two unlike charges. Many polyelectrolytes have high linear charge densities and are therefore likely to form strong complexes. In many cases these complexes form different forms of complex phase structure or morphology depending on many intrinsic and formulation variables including molecular weight, charge density and chemical structures of polymers or stoichiometry of the two polymers with opposite charges or concentration or them. By controlling these variables, different complex phases can he formed such as coacervate. water soluble complex, precipitate, micelles, gel matrix structure and microgel. Once polyelectrolyte complexes are formed, they represent unusual features of the polymeric materials that are completely different from their individual polymers before they form complexes. It has been found diverse uses of such complexes in pharmaceutical applications, cosmetics, coating, papermaking and numerous other related field of applications.

A polyelectrolyte complex (PEC) is formed herein between two polymers interacting through non-covalent bonding *e.g.* ionic, macro-ionic, hydrogen, electrostatic attraction or an associative mechanism of hydrophobic groups on the molecule. In the preferred example herein, two oppositely charged polymers interact through their cationic and anionic charges and/or an associative mechanism of hydrophobic groups on the molecules to form a polyelectrolyte complex in the form of a gel matrix structure.

In the present invention, the inventors surprisingly discovered a polyelectrolyte complex (PEC) in the form of gel matrix complex morphology formed between a cationic polymer and an anionic polymer with a defined charge density and charge ratio. Application of such complexes in the form of hair care compositions to the hair fibers and the method of use is capable of providing durable hair styling and/or aqueous wash-resistant retention of shape of hair fibers. Additionally, the durable styling benefit can last for at least about 2 shampoo wash cycles and/or conditioner rinse cycles, up to 5 wash cycles or more without further treating the hair fibers with hair care composition comprising polyelectrolyte complex. Therefore, it is significantly advantageous for the consumers in terms of economical benefit and use convenience with just single application to achieve styling effect that lasts a number of shampoo wash cycles and /or multiple conditioner rinsing.

What is described herein is an aqueous hair care composition which is capable of providing durable non-permanent hair styling and/or wash-resistant retention of shape of hair fibers comprising a polyelectrolyte complex having a gel matrix structure or a mixture of gel matrix and microgel formed by electrostatic attraction and/or an associative mechanism of hydrophobic groups on the molecules, wherein the polyelectrolyte complex **is as defined in claim 1.**

The hair care composition comprising gel matrix, or mixture of gel matrix and microgel is formed by mixing about 0.01 wt. % to about 20 wt. % of at least one anionic polymer selected from (A) and about 0.1 wt. % to about 20 wt. % of at least one

cationic polymer selected from (B) at room temperature, wherein A is added to B or B is added to A. and wherein said A is pre-neutralized to a pII of about 5 to about 10 before mixing with B.

The average molecular weight of the anionic polymer (A) of the present application is from about 100,000 Daltons to about 1000,000 Daltons; or about 1000.000 Daltons to about 2000,000 Daltons; or about 2000.000 Daltons to about 3000.000 Daltons: or about 3000.000 Daltons to about 4000,000 Daltons; or about 4000,000 Daltons to about 5000.000 Daltons. The preferred molecular weight of the anionic polymer (A) is from about 800.000 to 2.000,000 Daltons. The preferred quantity of anionic polymer range is from about 0.01 wt. % to about 5.0 wt. %; or about 5.0 wt. % to about 10.0 wt. %; or about 10.0 wt. % to about 15.0 wt. %; or about 15.0 wt. % to about 20.0 wt. %.

The average molecular weight of the cationic polymer (B) of the present application is from about 80,000 Daltons to about 1000,000 Daltons; or about 1000,000 Daltons to about 2000.000 Daltons; or about 2000.000 Daltons to about 3000.000 Daltons; or about 3000.000 Daltons to about 4000,000 Daltons; or about 4000,000 Daltons to about 5000,000 Daltons. The preferred molecular weight of the cationic polymer (B) is from about 200,000 to 2,000,000 Daltons. The preferred molecular weight of the cationic polymer (B) is from about 200,000 to 3,000,000 Daltons. The preferred quantity of anionic polymer range is from about 0.01 wt. % to about 5.0 wt. %; or about 5.0 wt. % to about 10.0 wt. %; or about 10.0 wt. % to about 15.0 wt. %; or about 15.0 wt. % to about 20.0 wt. %.

According to one embodiment of the present application, the charge ratio of cationic polymer (B) to anionic polymer (A) is more than about 0.70 and more preferably the charge ratio of B to A is above 1.0.

The hair styling composition of the present application comprises polyelectrolyte complex having (i) a gel matrix structure or (ii) a mixture of gel matrix and microgel is formed by electrostatic attraction and or associative mechanism of hydrophobic groups, and wherein, the % ratio of gel matrix and microgel formed or present in the mixture of gel matrix and microgel portion may comprise about 10% of microgel to about 90% of gel matrix or about 90% of microgel to about 10% of gel matrix. Gel matrix and microgel have different average particle sizes. Gel matrix has an average particle size of more than about 15 microns while microgel has an average particle size of less than about 7 microns. Particle size distribution of gel matrix and microgel mixture of the complex in water can be measured using Malvern Mastersizer. It helps to determine the particle size distribution of liquid dispersions by using Mie laser light scattering theory. According to important embodiment of the application, at least 10% of gel matrix is necessary to deliver the desired durable styling benefit of the hair care or hair styling composition of the present application. The presence of higher quantity of gel matrix would deliver higher styling benefit to the end-users.

According to another embodiment of the present application, the hair care composition comprising polyelectrolyte complex (PEC) containing the gel matrix structure is able to form a wash -resistant polymeric resinous film on the surface of hair upon atmosphere drying therefore provides advantageous hair shape benefits such as styleability, durability of style, anti-frizz, and/or hair manageability, and wherein, the type of hair care composition can be either a rinse-off or leave-in products. These hair shape benefits are significantly enhanced with the subsequent heating treatment of PEC treated hair. The preferred heating temperature for hair fibers is of about 90°C or more.

It is also demonstrated that the polyelectrolyte complex will contribute to style memory, which may allow hair to be re-styled to its original configuration with case. It also provides shine, wet conditioning, dry conditioning, ease of wet combing, hair protection from styling appliance.

Another embodiment of the present application discloses application of hair care composition to the desired hair fibers and followed by drying of the hair and subsequent heat treatment has provided significant advantages such as aqueous wash-resistant durable styling effect that can last multiple shampoo wash cycles and/or several conditioner rinse cycles with just single application of hair care composition comprising polyelectrolyte complex.

**The anionic polymer (A)** is a diacid or half-ester of methyl vinyl ether/ maleic anhydride copolymer (*i.e.*, a polymer from the Gantrez® family, i.e. Gantrez® S-97 Ashland Inc.).

The cationic polymer (B) is a polymer having an INCI designation of polyquaternium,

Polyquaternium polymers which are widely known as "PQ" polymers that comprise a quaternium ammonium moiety, and many such polymers are known in the prior art for a person skilled in the related art. For example, PQ-1 to PQ-47 polymers are duly listed in the Official Journal of the European Union, Commission Decision dated 09 February 2006. 2006/257/EC.Other quaternary ammonium compounds are listed in the CTFA Cosmetic Ingredient Handbook, First Edition, on pages 41-42, incorporated herein by reference, and are described in the "History of Polymers in Haircare." Cosmetics and Toiletries, 103 (1988), incorporated herein by reference.

Further, it is contemplated to employ **the** : polyquaternium polymers that may be employed in present application as cationic polymer and those are presented in the below **Table 1.**

**Table 1**: Polyquaternium polymers as cationic polymer **(B)**

| INCI name | Trade name | Manufacturer |
|-----------|------------|--------------|
| PQ-55 | Styleze® W | ASI |

Polyelectrolyte complexes of the present invention are made by the interacting at least two oppositely charged polymers or macromolecules. Polyelectrolyte complexes may be made from polymers with specific intrinsic variables (molecular weight, charge density, functional groups) as well as the right formulation variables (weight, ratio, solvent) to form a gel matrix structure or a mixture of gel matrix and microgel. These are dispersed phases that are dispersible in water and form a wash-resistant film upon drying and/or heating treatment.

One important example of a cationic/anionic polymer polyclectrolyte complex of the present application is a complex of a diacid or half ester of methyl vinyl either/ maleic anhydride coplolymer (Gantraze® S-97) and Polyquaternium® 55. At a defined preferred cationic/anionic ratio, the polyelectrolyte complex is present as major gel matrix structures and illustrated in Examples 1-3.

According to another embodiment of the present application, the hair care compositions may comprise one or more polyelectrolyte complexes that are disclosed in the present application.

According to one important aspect or embodiment of the present application, the hair care composition comprising polyelectrolyte complexes of the present application may be formulated as rinse-off or leave-in type of products. The non-limiting examples of rinse-off products include shampoos, conditioners, hair straighteners. permanent waves, and hair colors (encompassing permanent, semi-permanent, and temporary hair colors). Leave-in type of hair care products include hut not limited to the following representative examples such as hair sprays, mousses, hair lacquers, hair gels, hair waxes, styling creams, pomades, and tonics. Mention is made that the term "hair spray." as used herein. refers to hair care products that are delivered in any atomized (spray) format, whether they be pressurized or unpressurized.

It is also considered to employ polyelectrolyte complexes of the present application in the following non-limiting type of hair care and/or hair styling based end-user formulations such as 2 in 1 shampoos, leave in and rinse off conditioners, hair perming products, hair relaxants, permanent hair dyeing systems, hair styling mousses, semi-permanent hair dyeing systems, temporary hair dyeing systems, hair bleaching agents, permanent hair wave systems, hair setting formulations, non-coloring hair preparations, hair-frizz-control gels, hair leave-in conditioners, hair de-tangling products, hair fixatives, hair conditioning mists, hair care pump sprays and other non-aerosol sprays, hair cuticle coats.

It is contemplated but not limited to employ a heating treatment through hot flat iron, a curling iron or a hair blow drier which is specifically suggested to use on the PEC treated hair fibers to enhance the wash resistant feature of PEC film and provide durable styling benefit which can last over multiple washes and rinse cycles.

The end-user based rinse-off and leave-in type of products may comprise desired amount of the polyelectrolyte complex to attain desired benefit. In one embodiment, the addition level of the hair care composition comprising a polyelectrolyte complex in to the end-used products is ranging from about 0.1% to 10% by weight (with respect to the active content), and more particularly from about 0.25% to 8%. In certain embodiments of the invention, the addition level of the polyelectrolyte complex ranging from about 1% to about 3% by weight of the total composition. One skilled in the art would readily understand how to determine the required level hair composition of the present application according to the type of end-user product, for example, to optimize product performance.

The hair care compositions embraced by the invention deliver a multitude of durable hair care benefits. Since the polyelectrolyte complex (PEC) with the gel matrix structure is able to form a wash-resistant polymeric resinous film on the surface of hair upon drying and heating treatment, it provides hair shape benefits such as styleability, durability of style, anti-frizz, manageability from a rinse-off application and leave-in type products. These hair shape benefits are significantly enhanced with the subsequent heating treatment of PEC treated hair. It is also demonstrated that the polyelectrolyte complex will contribute to style memory, which may allow hair to be re-styled to its original configuration with ease. It also provides shine, wet conditioning, dry conditioning, case of wet combing, hair protection from styling appliance.

The hair care composition may contain at least one acceptable additive including but not limited to thickeners, viscosifiers, surfactants, cationic surfactants, emulsifiers, perfumes, preservatives, UV protectants, chelating agents, cleansing agents, wetting agents, hair conditioning ingredients, flexibility enhancers, split modifiers, conditioning ingredients, humectants, propellants, compressed gases, proteins, amino acids, shine enhancers, neutralizing agents, texturizing agents, water-proofing agents, solubilizers, suspension agents, suspended materials, cosmetically active ingredients, hair polymers, silicons, silicone-containing polymers, light protection agents, bleaches, gel formers, care agents, colorants, tinting agents, tanning agents, dyes, pigments, bodying agents, moisturizers, refatting agents, collagen, protein/keratin hydrolyzates, lipids, antioxidants, defoamers, antistatics, emollients, softeners, anti-dandruff agents, hair growth agents, antiinflammatory agents, anti-microbial agents, foam stabilizers, rheology modifiers, water softening agents, hydrotropes, polyalkylene glycols, acids, bases, buffers and blends thereof.

The hair conditioners include conditioning surfactants which are cosmetically acceptable and suitable for topical application to the hair. The preferred conditioning agent for the hair conditioner based composition is well for a person skilled in the relevant field. An extensive discussion on conditioning agents may be found in "Conditioning Agents for Skin and Hair", Cosmetic Science and Technology Series, Volume 21, 1999, Marcel Dekker Publishers. The contents of the book are hereby incorporated in its entirety by reference.

The conditioning agent can be a protein or hydrolyzed cationic or non-cationic protein. Examples of these compounds include hydrolyzed collagens having trimethyl ammonium and trimethyl stearyl ammonium chloride groups, hydrolyzed animal proteins having trimethyl benzyl ammonium groups (benzyltrimonium hydrolyzed animal protein), hydrolyzed proteins having groups of quaternary ammonium on the polypeptide chain, including at least one $C_1$-$C_{18}$ alkyl. Ceramide type of compound include a ceramide, a glycoceramide, a pseudoceramide, or a neoceramide. Possible ceramide type compounds useful herein include 2-N-linoleoyl amino-octadecane-1,3-diol, 2-N-oleoyl amino-octadecane-1,3-diol, 2-N-palmitoyl amino-octadecane-1,3-diol, 2-N-stearoyl amino-octadecane-1,3-diol, 2-N-behenoyl amino-octadecane-1,3-diol, 2-N-[2-hydroxy-palmitoyl]-amino-ocladecane-1,3-diol, 2-N-stearoyl amino-octadecane-1,3,4-triol. N-stearoyl phytosphingosine, 2-N-palmitoyl amino-hexadecane-1,3-diol, bis-(N-hydroxy ethyl N-cetyl) malonamide, N(2-hydroxy ethyl)-N-(3-cetoxyl-2-hydroxy propyl) amide of cetylic acid. N-docosanoyl N-methyl-D-glucamine and mixtures of such compounds.

Other types of conditioning agents can be a cationic surfactant such as a salt of a primary, secondary, or tertiary fatty amine, optionally polyoxyalkylenated, a quaternary ammonium salt, a derivative of imadazoline, or an amine oxide. Suitable examples include mono-, di-, or tri- alkyl quaternary ammonium compounds with a counterion such as a chloride, methosulfate, tosylate, etc. including, but not limited to, cetrimonium chloride, dicetyldimonium chloride, behentrimonium methosulfale, and the like. The presence of a quaternary ammonium compound in conjunction with the resin described above reduces static and enhances combing of hair in the dry state. The resin also enhances the deposition of the quaternary ammonium compound onto the hair substrate thus enhancing the conditioning effect of hair.

The conditioning agent can be any fatty amine known to be useful as a conditioning agent; e.g. dodecyl, cetyl or stearyl amines, such as stearamidopropyl dimethylamine.

The conditioning agent can be a fatty acid or derivatives thereof known to be useful as conditioning agents. Suitable fatty acids include myristic acid, palmitic acid, stearic acid, behenic acid, oleic acid, linoleic acid, and isostearic acid. The derivatives of fatty acids include carboxylic ester acids including mono-, di-, tri- and tetra- carboxylic acids.

Important non-limiting examples of certain cationic surfactants include: quaternary ammonium hydroxides and salts thereof, for example cetyl trimethylammonium chloride, stearyl dimethylbenzyl ammonium chloride, cetylpyridinium chloride, quatemium-5, quartemium-31, quarternium-18 and mixtures thereof.

Emulsifiers are selected from the group consisting of condensation products of aliphatic ($C_8$ to $C_{40}$) primary or secondary linear or branched-chain alcohols or phenols with alkylene oxides, $C_6$-$C_{100}$ ethylene oxide (EO), glycol distearate, sorbitan trioleate, propylene glycol isostearate, glycol stearate, sorbitan sesquioleate, glyceryl stearate, lecithin, sorbitan oleate, sorbitan monostearate NF, sorbitan stearate, sorbitan isostearate, steareth-2, oleth-2, glyceryl laurate, ceteth-2, PEG-30 dipolyhydroxystearale, glyceryl stearate SE, sorbitan stearate, sucrose cocoate, PEG-4 dilaurate, methyl glucose sesquistearate, PEG-8 dioleate, sorbitan laurate, PEG-40 sorbitan peroleate, laureth-4, PEG-7 glyceryl cocoate, PEG-20 almond glycerides, PEG-25 hydrogenated castor oil, stearamide MEA, glyceryl stearate, PEG-100 stearate, polysorbate 85, PEG-7 olivate, cetearyl glucoside, PEG-8 oleate, polyglyceryl-3 methyglucose distearate, Oleth-10. Oleth-10 / polyoxyl 10 oleyl ether NF, ceteth-10, PEG-8 laurate, cocamide MEA, polysorbate 60 NF, polysorbate 60, polysorbate 80, isosteareth-20, PHG-60 almond glycerides, polysorbate 80 NF, PEG-20 methyl glucose sesquistearate, ceteareth-20, oleth-20, steareth-20, steareth-21, ceteth-20, isoceteth-20, polysorbate 20, polysorbate 20 NF, laureth-23, PEG-100 stearate, steareth-100, PEG-80 sorbitan laurate alone or in combinations thereof.

It is contemplated to employ suitable silicones for preparing hair styling composition of the present application. The preferred silicone of the present application may be selected from the group consisting of but not limiting to polymethyl siloxane, polydimethyl siloxane/methyl vinyl siloxane gums, polydimethyl siloxane/diphenyl siloxane, polydimethyl siloxane/phenyl methyl siloxane and polydimethyl siloxane/diphenyl siloxane/methyl vinyl siloxanes, organo-modified silicone, amino functional silicones. The silicones may be used in the form of emulsions, nano-emulsions, or micro-emulsions. Further, the possible other silicones that are disclosed in WO2013064596A1 is considered for the purposes of the application and is enclosed here in its entirety.

The hair care compositions according to the present application may also comprise thickeners and/or viscosifiers from the following commercial products:

(1) Aqualon™ carboxymethylcellulose. Benecel™ methylcellulose and hydroxypropyl methylcellulose, Blanose™ sodium carboxymethylcellulose, Klucel™ hydroxypropylcellulose, Natrosol™ hydroxyethylcellulose, Natrosol™ Plus and PolySurf™ cetyl modified hydroxyethylcellulose, N-Hance™ cationic guar. N-Hance™ HP Series hydroxypropyl guar. N-Hance™ SP-100 conditioning polymer, and Supercol™ guar gum.

(2) Carbopol® Polymers, Fixate™ PLUS Polymer, Glucamate™ Thickeners. Amidex™ Surfactants, Chembetaine™ Surfactants, Chemoxide™ Surfactants. Chemonic™ Surfactants, Chemccinate™ Surfactants, Amidex™ BC-24 Surfactant, Chemoryl™ LB-30 Surfactant, Novethix™ L-10 Polymer, Ceralan™ Lanolin Product. Pemulen™ TR-1 Polymeric Emulsifier. Pemulen™ TR-2 Polymeric Emulsifier. Hydramol™ PGPD Ester. Schercodine™ M Amido-Amine, Sehereodine™ P Amido-Amine, Schercomid™ Diethanolamides from The Lubrizol Corporation.

(3) Salcare® and Luvigel® from BASF Corporation.

(4) Aculyn™ 22, Aculyn™ 28. Aculyn™ 33, Aculyn™ 38, and Aculyn™ 44 from The Dow Chemical Company.

(5) Amnionyx® C and Stepan-Mild® GCC from Stepan Company.

(6) Stabileze®, Rapithix® A-60. Rapithix® A-100, Ultrathix® P-100, Lubrajel® and FlexiThix from ASI.

Also suitable as a thickener/rheology modifier are lightly to moderately crosslinked polyvinylpyrrolidones. Disclosures of these polymers are provided in the following patents and publications, each of which is hereby incorporated in its entirety by reference: U.S. Patent 5.073,614; 5,312,619; 5,139,770; 5,716.634; 5.470.884; 5,759,524; 5,997,887; 6,024,942; and WIPO PCT publication numbers PCT/US10/26973, PCT/US10/26976, PCT/US10/26940, PCT/US 11/32993. and PCT/US11/34515.

The hair care compositions of the present application may also comprise one or more thickening polymers. The Non-limiting examples of suitable thickening polymers include: Acetamide MEA; acrylamide/ethalkonium chloride acrylate copolymer; acrylamide/ethyltrimonium chloride acrylate/ethalkonium chloride acrylate copolymer; acrylamides copolymer; acrylamide/sodium acrylate copolymer; acrylamide/sodium acryloyldimethyltaurate copolymer; acrylates/acetoacetoxyethyl methacrylate copolymer; acrylates/beheneth-25 methacrylate copolymer; acrylates/C10-C30 alkyl acrylate crosspolymer: acrylates/ceteth-20 itaconate copolymer: acrytates/ceteth-20 methacrylate copolymer: acrylates/laureth-25 methacrylate copolymer; acrylates/palmeth-25 acrylate copolymer; acrylates/palmeth-25 itaconate copolymer; acrylates/steareth-50 acrylate copolymer; acrylates/steareth-20 itaconate copolymer; acrylates/steareth-20 methacrylate copolymer; acrylates/stearyl methacrylate copolymer; acrylates/vinyl isodecanoate crosspolymer; acrylic acid/acrylonitrogens copolymer; adipic acid/methyl DEA crosspolymer; agar; agarose; alcaligenes polysaccharides: algin: alginic acid; almondamide DIA; almondamidopropyl betaine; aluminum/magnesium hydroxide stearate: ammonium acrylates/acrylonitrogens copolymer; ammonium acrylates copolymer; ammonium acryloyldimethyltaurate/vinyl formamide copolymer; ammonium acryloyldimethyltaurate/VP copolymer; ammonium alginate; ammonium chloride: ammonium polyacryloyldimethyl taurate; ammonium sulfate; amylopectin; apricolamide DEA; apricotamidopropyl betaine; arachidyl alcohol; arachidyl glycol; arachis hypogaea (peanut) flour; ascorbyl methylsilanol pectinate; astragalus gummifer gum; attapulgite; avena sativa (oat) kernel flour; avocadamide DEA; avocadamidopropyl betaine; azelamide MEA; babassuamide DEA; babassuamide MEA; babassuamidopropyl betaine; behenamide DEA; behenamide MEA; behenamidopropyl betaine; behenyl betaine; bentonite; butoxy chitosan: caesalpinia spinosa gum; calcium alginate: calcium carboxymethyl cellulose; calcium carrageenan: calcium chloride; calcium potassium carbomer; calcium starch octenylsuccinate; C20-40 alkyl stearate; canolamidopropyl betaine; capramide DEA; capryl/capramidopropyl betaine; carbomer; carboxybutyl chitosan; carboxymethyl cellulose acetate butyrate; carboxymethyl chitin; carboxymethyl chitosan; carboxymethyl dextran; carboxymethyl hydroxyethylcellulose; carboxymethyl hydroxypropyl guar; carnitine; cellulose acetate propionate carboxylate: cellulose gum; ceratonia siliqua gum; cetearyl alcohol; cetyl alcohol; cetyl babassuate; cetyl betaine; cetyl glycol: cetyl hydroxyethylcellulose; chimyl alcohol; cholesterol/HDI/pullulan copolymer; cholesteryl hexyl dicarbamate pullulan: citrus aurantium dulcis (orange) peel extract; cocamide DHA; cocamide MEA; cocamide MIPA; cocamidoethyl betaine; cocamidopropyl betaine; cocamiclopropyl hydroxysultaine; coco-betaine; coco-hydroxysultaine; coconut alcohol; coco/oleamidopropyl betaine; coco-Sultaine; cocoyl sarcosinamide DEA; comamide/cocamide DEA; cornamide DEA; croscarmellose; crosslinked bacillus/glueose/sodium glutamate ferment; cyamopsis tetragonoloba (guar) gum; decyl alcohol; decyl betaine; dehydroxanthan gum; dextrin: dibenzylidene sorbitol: diethanolaminooleamide DEA; diglycol/CHDM/isophthalates/SIP copolymer; dihydroabietyl behenate; dihydrogenated tallow benzylmonium hectorite; dihydroxyaluminum aminoacetate; dimethicone/PHG-10 crosspolymer; dimethicone/PEG-15 crosspolymer; dimethicone propyl PG-betaine; dimethylacrylamide/acrylic acid/polystyrene ethyl methacrylate copolymer; dimethylacrylamide/sodium acryloyldimethyltaurate crosspolymer; disteareth-100 IPDI; DMAPA acrylates/acrylic acid/acrylonitrogens copolymer; erucamidopropyl hydroxysultaine; ethylene/sodium acrylate copolymer: gelatin; gellan gum; glyceryl alginate; glycine soja (soybean) flour; guar hydroxypropyltrimonium chloride; hectorite; hyaluronic acid; hydrated silica; hydrogenated potato starch; hydrogenated tallow; hydrogenated tallowamide DEA; hydrogenated tallow betaine; hydroxybutyl methylcellulose; hydroxyethyl acrylate/sodium acryloyldimethyl taurate copolymer; hydroxyethylcellulose; hydroxyethyl chitosan; hydroxyethyl ethylcellulose; hydroxyethyl stearamide-MIPA; hydroxylauryl/hydroxymyristyl betaine; hydroxypropylcellulose; hydroxypropyl chitosan; hydroxypropyl ethylenediamine carbomer; hydroxypropyl guar; hydroxypropyl methylcellulose; hydroxypropyl methylcellulose slearoxy ether; hydroxypropyl starch; hydroxypropyl starch phosphate; hydroxypropyl xanthan gum; hydroxystearamide MEA; isobutylene/sodium maleate copolymer; isostearamide DEA; isostearamide MEA; isostearamide mIPA; isostearamidopropyl betaine; lactamide MEA; lanolinamide DEA; lauramide DEA; lauramide MEA. lauramide MIPA; lauramide/myristamide DEA; lauramidopropyl betaine; lauramidopropyl hydroxysultaine: laurimino bispropanediol; lauryl alcohol; lauryl betaine; lauryl hydroxysultaine; lauryl/myristyl glycol hydroxypropyl ether; lauryl sultaine; lecithinamide DEA; linoleamide DEA; linoleamide MEA; linoleamide MIPA; lithium magnesium silicate; lithium magnesium sodium silicate; macrocystis pyrifera (kelp); magnesium alginate; magnesium/aluminum/hydroxide/carbonate; magnesium aluminum silicate; magnesium silicate; magnesium trisilicate; methoxy PEG-22/dodecyl glycol copolymer; methylcellulose; methyl ethylcellulose; methyl hydroxyethylcellulose; microcrystalline cellulose: milkamidopropyl betaine; minkamide DEA; minkamidopropyl betaine; MIPA-myristate; montmorillonite; Moroccan lava clay; myristamide DEA; myristamide MEA; myristamide MIPA; myristamidopropyl betaine: myrislamidopropyl hydroxysultaine; myristyl alcohol; myristyl betaine; natto gum; nonoxynyl hydroxyethylcellulose; oatamide MEA; oatamidopropyl betaine; octacosanyl glycol isostearate; octadecene/MA copolymer; oleamide DEA; oleamide MEA; oleamide MIPA; oleamidopropyl betaine; oleamidopropyl hydroxysultaine; oleyl betaine; olivamide DEA; olivamidopropyl betaine; oliveamide MEA; palmamide DEA; palmamide MEA; palmamide MIPA; palmamidopropyl betaine; palmitamide DEA; palmitamide MEA; palmitamidopropyl hetaine; palm kernel alcohol; palm kernelamide DEA; palm kernelamide MEA; palm kernelamide MIPA; palm kernelamidopropyl betaine; peanutamide MEA; peanulamide MIPA; pectin; PEG-800; PEG-crosspolymer; PEG-150/decyl alcohol/SMDI copolymer; PEG-175 diisostearate; PEG-190 distearate; PEG-15 glyceryl tristearate; PEG-140 glyceryl tristearate; PEG-240/IIDI copolymer bis-decyltetradeceth-20 ether; PEG-100/IPDI copolymer; PEG-180/laureth-50/™MG copolymer; PEG-10/lauryl dimethicone crosspolymer; PEG-15/lauryl dimethicone crosspolymer; PEG-2M; PEG-5M; PEG-7M; PEG-9M; PEG-14M; PEG-20M; PEG-23M; PEG-25M; PEG-45M; PEG-65M; PEG-90M; PEG-115M; PEG-160M; PEG-180M; PEG-120 methyl glucose trioleate; PEG-180/octoxynol-40/™MG copolymer; PEG-150 pentaerythrityl tetrastearate; PEG-4 rapeseedamide; PEG-150/stearyl alcohol/SMDI copolymer: phaseolus angularis seed powder; polianthes tuberosa extract; polyacrylate-3; polyacrylic acid; polycyclopentadiene; polyetherl; polyethylene/isopropyl maleate/MA copolyol; polyglyceryl-3 disiloxane dimethicone; polyglyceryl-3 polydimethylsiloxyethyl dimethicone; polymethacrylic acid; polyquaternium-52; polyvinyl alcohol; potassium alginate; potassium aluminum polyacrylate; potassium carbomer; potassium carrageenan; potassium chloride; potassium palmate; potassium polyacrylate: potassium sulfate; potato starch modified; PPG-2 cocamide: PPG-1 hydroxyethyl caprylamide; PPG-2 hydroxyethyl cocamide; PPG-2 hydroxyethyl coco/isostearamide; PPG-3 hydroxyethyl soyamide; PPG-14 laureth-60 hexyl dicarbamate; PPG-14 laureth-60 isophoryl dicarbamate; PPG-14 palmeth-60 hexyl dicarbamate; propylene glycol alginate; PVP/decene copolymer; PVP montmorillonite; pyrus cydonia seed; pyrus malus (apple) fiber, rhizobian gum; ricebranamide DEA; ricinoleamide DEA; ricinoleamide MEA; ricinoleamide MIPA; ricinoleamidopropyl betaine; ricinoleic acid/adipic acid/AEEA copolymer; rosa multiflora flower wax: sclerotium gum; sesamide DEA; sesamidopropyl betaine; sodium acrylate/acryloyldimethyl taurate copolymer; sodium acrylates/acrolein copolymer; sodium acrylates/acrylonitrogens copolymer: sodium acrylates copolymer; sodium acrylatcs crosspolymer: sodium acrylate/sodium acrylamidomethylpropane sulfonate copolymer: sodium acrylates/vinyl isodecanoate crosspolymer; sodium acrylate/vinyl alcohol copolymer; sodium carbomer; sodium carboxymethyl chitin; sodium carboxymethyl dextran; sodium carboxymethyl beta-glucan; sodium carboxymethyl starch; sodium carrageenan; sodium cellulose sulfate; sodium chloride;

sodium cyclodextrin sulfate; sodium hydroxypropyl starch phosphate; sodium isooctylene/MA copolymer; sodium magnesium fluorosilicate; sodium oleate; sodium palmitate; sodium palm kernelate; sodium polyacrylate; sodium polyacrylate starch; sodium polyacryloyldimethyl taurate: sodium polygamma-glutamate; sodium polymethacrylate; sodium polystyrene sulfonate; sodium silicoaluminate; sodium starch octenylsuccinate; sodium stearate; sodium stearoxy PG-hydroxyethylcellulose sulfonate; sodium styrene/acrylates copolymer; sodium sulfate; sodium tallowate; sodium tauride acrylates/acrylic acid/acrylonitrogens copolymer; sodium tocopheryl phosphate; solanum tuberosum (potato) starch; soyamide DEA; soyamidopropyl betaine; starch/acrylates/acrylamide copolymer; starch hydroxypropyltrimonium chloride: stearamide AMP: stearamide DEA: stearamide DEA-distearate: stearamide DIBA-stearate; stearamide MEA; stearamide MEA-stearate: stearamide MIPA; stearamidopropyl betaine; steareth-60 cetyl ether; steareth-100/PEG-136/HDI copolymer; stearyl alcohol; stearyl betaine; sterculia urens gum; synthetic fluorphlogopite; tallamide DEA; tallow alcohol; tallowamide DEA; tallowamide MEA; tallowamidopropyl betaine; tallowamidopropyl hydroxysultaine; tallowamine oxide; tallow betaine; tallow dihydroxyethyl betaine; tamarindus indica seed gum; tapioca starch; TEA-alginate; TEA-carbomer; TEA-hydrochloride; trideceth-2 carboxamide MEA; tridecyl alcohol; methylene glycol dibenzoate; trimethyl pentanol hydroxyethyl ether; triticum vulgare (wheat) germ powder; triticum vulgare (wheat) kernel flour; triticum vulgare (wheat) starch; tromethamine acrylates/acrylonitrogens copolymer; tromethamine magnesium aluminum silicate; undecyl alcohol; undecylenamide DEA; undecylenamide MEA; undecylenamidopropyl betaine; welan gum; wheat germamide DEA; wheat germamidopropyl betaine; xanthan gum; yeast beta-glucan; yeast polysaccharides; zea mays (corn) starch; and blends thereof.

The hair care composition of present application can be preserved by adding minor quantity of preservatives to the compositions. Such preservatives can be selected from, but are not limited to, triazoles, imidazoles, naphthalene derivatives, benzimidazoles, morphline derivatives, dithiocarbamates, benzisothiazoles, benzamides, boron compounds, formaldehyde donors, isothiazolones, thiocyanates, quaternary ammonium compounds, iodine derivates, phenol derivatives, Phenoxyethanol and Caprylyl Glycol micobicides, pyridines, dialkylthiocarbamates, nitriles, parabens, alkyl parabens and salts thereof.

Suitable antioxidants may be added to facilitate the enhanced shelf-life of the hair styling composition of the present application. Exemplary antioxidants that can be used include vitamins such as vitamin E, vitamin E acetate, vitamin C, vitamin A, and vitamin D. and derivatives thereof. Additional exemplary antioxidants include but are not limited to propyl, octyl and dodecyl esters of gallic acid, butylated hydroxyanisole (BHA), butylated hydroxytoluene (BHT), and nordihydroguaiaretic acid. In general, the required amount of antioxidant for the present composition is in the range of about 0.2 wt. % to about 2 wt. %, and can be provided in an amount of about 0.5 wt. % to about 1.5 wt. %, based on the total weight of the composition.

The preferred fatty substance based excipient for formulating desired hari styling composition of the present application would include fatty alcohols, natural and synthetic waxes, ceramides. mineral oils, vegetable oils, animal oils, synthetic oils. The other preferred fatty substance are isododecane, hydrogenated polyisobutene, squalane, isononyl isononanoate, cyclotetra- and - pentadimethicones, phenyltrimethicone, ethylene homopolymers, ethoxylated fats and oils, fluoroalkanes, seracite, shea butter, arachidyl propionate alone or in combination. For the definition of waxes, mention may be made, for example, of P.D. Dorgan, Drug and Cosmetic Industry, December 1983. pp. 30-33.

Moisturizers or humactant employed in the present invention would include glycols, glycerols, propylene glycol, diethylene glycol monoethyl ether, sorbitol, sodium salt of pyroglutamic acid, glycerol, glycerol derivatives, glycerin, trehalose, sorbitol, maltitol, dipropylene glycol, 1,3-butylene glycol, sodium hyaluronate, and the like.

Further, it is known that moisturizers that binds well with water, thereby retaining it on the hair surface are called humectants. Examples of humectants which can be incorporated into a product of the present application are glycerine, propylene glycol, polypropylene glycol, polyethylene glycol, lactic acid, sodium lactate, pyrrolidone carboxylic acid, urea, phospholipids, collagen, elastin, ceramides, lecithin sorbitol, PEG-4, and mixtures thereof. Additional suitable moisturizers are polymeric moisturizers that belong to water soluble and/or water swellable in nature. Polysaccharides such as hyaluronic acid, chitosan can also be employed along with moisturizers of the present application as binder to enhance their property.

Examples of anti-dandruff agents that can he used are cimbazole, octopirox and zinc pyrithione, salicylic acid, elemental sulfur, selenium dioxide, and the azole antimycotics.

According to one important embodiment of the present invention, it is contemplated to employ at least one organic UV filters which can filter out UV rays. The filter can be selected from hydrosoluble or liposoluble filters, whether siliconated or nonsiliconated, and mineral oxide particles, the surface of which may be treated. Hydrosoluble organic UV filters can be chosen from *para*-amino benzoic acid and its salts, anthranilic acid and its salts, salicylic acid and its salts, hydroxy cinnamic acid and its salts, sulfonic derivatives of benzothiazoles, benzimidizoles, benzoxazoles and their salts, sulfonic derivatives of benzophenone and their salts, sulfonic derivatives of benzylidene camphor and their salts, derivatives of benzylidene camphor substituted by a quaternary amine and their salts. derivatives of phthalydene-camphosulfonic acids and their salts, sulfonic derivatives of benzotriazole, and mixtures thereof.

Suitable liposoluble organic UV filters include, but not limited to, derivatives of para-aminobenzoic acid, such as the esters or amides of para-aminobenzoic acid; derivatives of salicylic acid; derivatives of benzophenone; derivatives of dibenzoyl methane; derivatives of diphenyl acrylates; derivatives of benzofurans; UV filter polymers containing one or more silico-organic residues; esters of cinnamic acid; derivatives of camphor; derivatives of trianilino-s-triazine; the ethylic ester urocanic acid; benzotriazoles; derivatives of hydroxy phenyl triazine; *bis*-resorcinol-dialkyl amino triazine; and mixtures thereof.

The coloring agents, colorants or dyes used herein include natural foods colors and dyes suitable for food. drug and cosmetic applications. These colorants are also known as FD & C, and D&C dyes and lakes and are preferably water-soluble in nature. A full recitation of all FD&C and D&C dyes and their corresponding chemical structures may be found in the Kirk-Othmer Encyclopedia of Chemical Technology, Volume 5, pages 857-884, which text is accordingly incorporated herein by reference. These coloring agents may be incorporated in amount up to about 3%, more particularly up to about 2%, and in some cases less than about 1% by weight of the personal care compositions.

The term "sequestering agent" or "chelating agent" as used herein relates to a compound which is capable of bonding or complexing a metal ion between two or more atoms of the compound, thereby neutralizing or controlling harmful effects of such metal ions, wherein holding or bonding of a metal ion is through combination of one or more different types of bonds including coordination and/or ionic bonds. The suitable organic or inorganic sequestering or chelating for the purposes of the present application is selected from the group comprising polyols, gluconates, sorbitals, mannitols, carbonates, hydroxamates, catechols, $\alpha$-amino carboxylates, alkanolamines, metal-ion sequestrants, hydroxy-carboxylic acids, aminocarboxylic acids, amino polycarboxylic acids, polyamines, polyphosphates, phosphonic acids, crown ethers, amino acids, polycarboxylic acids, cyclodextrin, phosphonates, polyacrylates or polymeric polycarboxylates, condensed phosphates. Further, the information on sequestering and chelating agents is disclosed in T. E. Furia, CRC Handbook of Food Additives, 2nd Edition, pp. 271-294 (1972), and M. S. Peterson and A. M. Johnson (Eds.), Encyclopedia of Food Science, pp. 694-699 (1978) are incorporated herein by reference in its entirety.

A perfume or fragrance obtained from natural or synthetic source can be employed in the present hair care composition. The fragrance may be used along with a suitable solvent, diluents or carrier. Fragrances may be added in any conventionally known method, for example, admixing to a composition or blending with other ingredients used to form a composition, in amounts which are found to be useful to increase or impart, the desired scent characteristics to the disinfectant or cleaning compositions. Fragrances for the present application can be one or more selected from the following non-limiting group of compounds such as essential oils, absolutes, resinoids, resins, concretes, hydrocarbons, alcohols. aldehydes, ketones, ethers, acids, esters, acetals, ketals, nitriles, including saturated and unsaturated compounds and aliphatic, carbocyclic and heterocyclic compounds.

The preferred neutralizing agents that can be employed for neutralizing anionic polymer A would include but not limited to alkali hydroxides such as a sodium and potassium hydroxide; organic bases such as methylethylamine (MEA), ammonia, aminoalcohols, lithium hydroxide, diethanolamine (DEA); triethanolamine (TEA), aminomethyl propanol, and mixtures thereof. The neutralizing agent can be present in an amount of about 0.01 *wt.* % to about 8 wt. %, preferably, 1 wt % to about 5 wt. %. The desired pH of anionic polymer $\Lambda$ for preparing hair styling composition is in the range of from about 6 to about 8, and in some embodiment, it is preferably about 7. The utility levels of the pH modifying agent may be present in an effective amount required to achieve the desired pH level.

The pH of the composition can be controlled within any desired range according to the type and purpose of the formulation. In order to attain the desired pH range, various pH modifiers may be employed in the present composition. Specific examples of basic pH modifiers are ammonia; sodium, potassium, and lithium hydroxide; sodium, potassium, and lithium meta silicates; monoethanolamine; triethylamine: isopropanolamine; diethanolamine; and triethanolamine. The suitable acidic pH modifying agents that can be employed in the present application include, but are not limited to, mineral acids, carboxylic acids and polymeric acids. Specific examples for mineral acids are hydrochloric acid, nitric acid, phosphoric acid, and sulfuric acid. Examples for appropriate carboxylic acids are citric acid, glycolic acid, lactic acid, maleic acid, malic acid, succinic acid, glutaric acid, benzoic acid, malonic acid, salicylic acid, gluconic acid, and mixtures thereof. Examples for suitable polymeric acids include straight-chain poly(acrylic) acid and its copolymers (e.g., maleic-acrylic, sulfonic-acrylic, and styrene-acrylic copolymers), cross-linked polyacrylic acids having a molecular weight of less than about 250,000, poly(methacrylic) acid, and naturally occurring polymeric acids such as carageenic acid, carboxymethyl cellulose, and alginic acid.

Methodology for assessing hair styling and fixative effect:

(1) Durable styling test with heating treatment:

    i. Wash tress with 12% SLES.
    ii. Apply 0.3g PEC formulation to a 2g hair tress.
    iii. Blow drying the tress after PEC treatment.

iv. Comb through the dried tress to make the hair fibers smooth before heating treatment.

v. Set hot flat iron to 205 °C to 232 °C or the highest temperature setting of flat iron.

vi. Hot flat ironing the dried PEC treated tress from top to the bottom at 5 seconds pace for each pass for 10 passes.

vii. Wash the hot flat ironed tress with 12% SLES again with 0.3ml of SLES for 1 minute and rinse for 30 seconds.

viii. Squeeze the tress with two fingers to get rid of the excess water and make the tress into a straight shape and smooth it, then hang it drying in the air without disturbing it until dry.

ix. Evaluate the stiffness or holding strength of the tress, high humidity curl retention and fiber alignment after air drying by comparing the control hair tress which is untreated but hot flat ironed.

x. Repeat step vii to ix up to 5 cycles.

(2) High humidity curl retention tests

The hair tresses from the PEC treatment following the steps described above were re-wet and rolled in wet with rollers. The rollers were dried overnight. The hair rolls are taken out and rollers are removed. The hair rolls are then placed in a high humidity chamber at 90% RII. The high humidity curl retention is measured over the time.

(3) Hair salon tests

Whole head tests in a hair salon were conducted on mannequins or on a live panelist with the inventive composition and the control by a professional cosmetologist for evaluating styling attributes such as styleability, curl definition, fiber alignment, frizz and hair manageability.

Methodology for assessing hair conditioning effect:

Combing force of hair tresses treated with the inventive composition and after shampoo washes was measured against control.

$$\text{Combing force reduction \%} = \frac{\text{Initial combing force} - \text{treated combing force}}{\text{Initial combing force}}$$

Further, the present invention is illustrated in detail by way of the below given examples. The examples are given herein for illustration of the invention and are not intended to be limiting thereof.

## EXAMPLES

### Examples 1 to 4

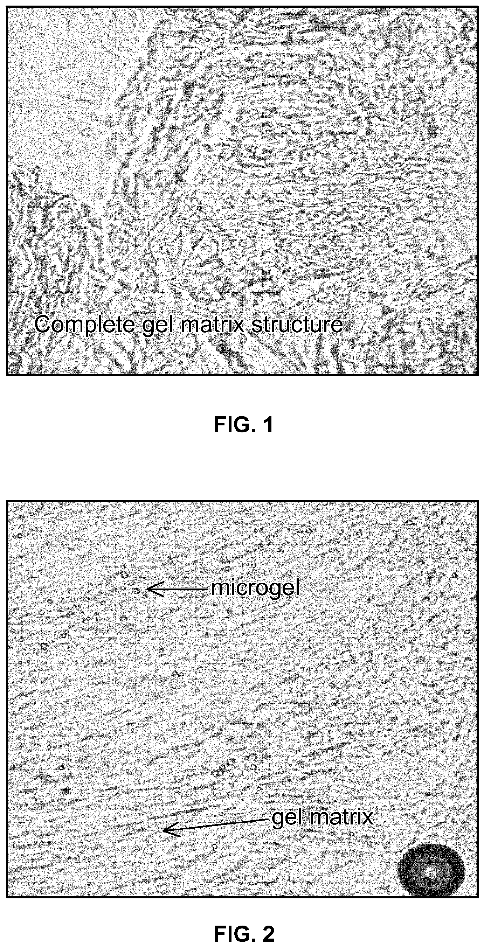

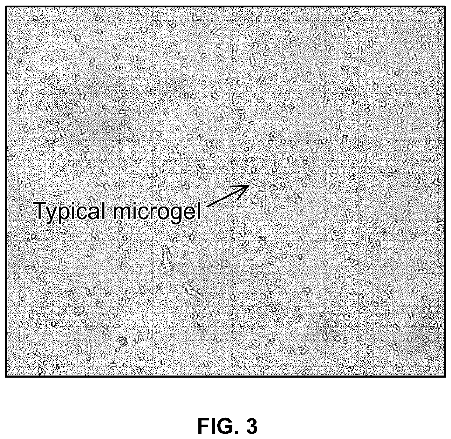

Table 1 A shows examples of polyelectrolyte complex (PEC) compositions having cationic to anionic charge ratio of from 0.6 to 2.26 which is within and outside the claim scope of this application. Wherein, Example 1, Example 2 are PEC made with polyquatrenium-55, a cationic polymer with a charge density of less than 2 and the charge ratios of cationic polymer to anionic polymer are more than 1. The complexes of Example 1 and 2 form a phase structure containing gel matrix and provide high extra holding strength on hair over the untreated control, whereas Example 3 made with the same polymers but has a charge ratio of 0.62 forms a typical microgel phase and has much lower holding strength on hair than the complex with a gel matrix structure in Example 1 & 2. Example 4 is a PEC made with a cationic polymer having a charge density higher than 4 which forms precipitate instead. Further. **Figure 1, 2, and 3** show the microscope images of polyelectrolyte complex phases at different charge ratios of the cationic polymer to the anionic polymer.

**Table 1A: PEC compositions versus theirs complex phase structure**

| Ingredients/Properties | EXP 1 pH 7 | EXP 2 pH 7 | EXP 3 pH 5.5 | EXP 4 pH 7 |
|---|---|---|---|---|
| | (Wt. %) | | | |
| Methylvinylether/maleic anhydride coplolymer (Anionic) | 0.45 | 0.728 | 0.728 | 1.0 |
| VP/DMAPA/C12-MAPTAC copolymer. 20% (Cationic) [Charge density <2] | 21.5 | 16.3 | 9.78 | - |
| PolyAPTAC [Charge density>4] | - | - | - | 2.6 |

(continued)

| Ingredients/Properties | EXP 1 pH 7 | EXP 2 pH 7 | EXP 3 pH 5.5 | EXP 4 pH 7 |
|---|---|---|---|---|
| | (Wt. %) | | | |
| Cationic to anionic charge ratio | 2.26 | 1.06 | 0.62 | 1.1 |
| Phase structure | All gel matrix | Major gel + some microgel | Microgel only | Precipitate |
| Extra holding strength over untreated* | 81% | 75% | 40% | N/A |

*The extra holding strength is measured hy holding the top of the treated, heated and washed and dried tress horizontally over a counter top and measuring the distance between the ends of the tress to the counter top and comparing that distance of an untreated control. The extra percent of distance over the control is calculated as the holding strength.

**Examples 5 to 12:** The Examples 5 to 12 in Table 2 are more Polyelectrolyte Complex (PEC) compositions those are within the scope of this invention.

**Table 2:** Concentrated PEC compositions

| Ingredients (INCI name) | Ex. 5 97-1 | Ex. 6 97-2 | Ex. 7 84-1 | Ex. 8 89-1 | Ex. 9 89-2 | Ex. 10 83-1 | Ex. 11 93-3 | Ex. 12 |
|---|---|---|---|---|---|---|---|---|
| | (Wt. %) | | | | | | | |
| Methylvinyl ether/maleic anhydride (MVE/MA) copolymer | 0.728 | 0.40 | 0.45 | - | - | 0.45 | - | 0.40 |
| Dodecyl vinyl ether/maleic acid copolymer, 5.11% | - | - | - | 8.80 | 8.80 | - | 8.80 | - |
| VP/DMAPA/C12-MAP TAC copolymer, 20% | 16.36 | - | - | - | 26.62 | 55.0 | - | - |
| PVP/DMAPA acrylate copolymer, 10% | - | 36.0 | 53.0 | 53.0 | - | - | - | - |
| PQ-28. 20% | - | - | - | - | - | - | - | 18 |
| PVP/TBAMEA, 16.8% | - | - | - | - | - | - | 51.5 | - |
| NaOH, 10%. | 2.18 | 1 | 1.35 | 0.57 | 0.5 | 1.10 | 1.10 | 1.20 |
| Water | BAL | BAL | BAL | BAL | BAL | BAL | BAL | BAL |
| Charge ratio of cationic to anionic polymer | 1.06 | 1.10 | 1.44 | 2.76 | 2.57 | 2.72 | 2.1 | 1.08 |
| PEC concentration | 4% | 4% | 5.75% | 5.75% | 5.75% | 5.77 | 9.11% | 4% |

DMAPA =dimethylaminopropyl methacrylamide: TBAME = tert-butylaminoethyl methacrylate

**Example 13:** This example shows style durability and conditioning effect of PECs treated hair fibers after one shampoo wash from a leave-in treatment followed with heating treatment. The High Humidity Curl Retention data shown in **Table 3** clearly shows that the PEC (from EX 5, 6 and 12) treated hair tresses with and without heating treatment have higher High Humidity Curl Retention than the control, untreated tress and also the tress treated with their non-complexed single polymers alone. In addition, heating treatment of PEC treated hair increases durable styling performance by showing higher High Humidity Curl Retention (HHCR).

**Table 3:** High Humidity Curl Retention (HHCR) of hair tresses after treatment with 2% PEC solution, dried, heated and washed one time with shampoo. Asian bleached hair

| S.No. | HHCR, POST-IX wash | | 0 | 30min. | 45min. | 60min. | 75min. |
|---|---|---|---|---|---|---|---|
| | | | Wt. % | | | | |
| 1. | Untreated | Heated | 100 | 33.04 | 21.74 | 18.26 | 16.52 |

(continued)

| S.No. | HHCR, POST-IX wash | | 0 | 30min. | 45min. | 60min. | 75min. |
|---|---|---|---|---|---|---|---|
| | | | Wt. % | | | | |
| 2. | Untreated | Not heated | 100 | 35.34 | 19.83 | 12.93 | 8.62 |
| 3. | 2% Methylvinyl ether/maleic anhydride (MVE/MA) copolymer, | Heated | 100 | 45.16 | 31.45 | 24.19 | 20.16 |
| 4. | 2% Methylvinyl ether/maleic anhydride copolymer | Not heated | 100 | 44.25 | 30.97 | 17.70 | 8.85 |
| 5. | 2% PVP/DMAPA acrylate copolymer | Heated | 100 | 84.75 | 69.49 | 59.32 | 51.69 |
| 6. | 2% PVP/DMAPA acrylate copolymer | Not heated | 100 | 59.09 | 31.82 | 18.18 | 9.09 |
| 7. | 2% PQ-55 | Heated | 100 | 62.3 | 50.00 | 46.15 | 38.46 |
| 8. | 2% PQ-55 | Not Heated | 100 | 39.17 | 29.17 | 20.83 | 8.33 |
| 9. | **EXP 12,** PEC. 2% MVE/MA copolymer and PQ-28 | Heated | 100 | 75.41 | 73.77 | 53.28 | 36.07 |
| 10. | **EXP 12,** PEC. 2% MVE/MA copolymer and PQ-28 | Not Heated | 100 | 49.11 | 32.14 | 22.32 | 17.86 |
| 11. | **EXP 6,** PEC, 2% MVE/MA copolymer & PVP/DMAPA | Heated | 100 | 88.80 | 76.00 | 68.00 | 51.20 |
| 12. | **EXP 6,** PEC, 2% MVE/MA copolymer & PVP/DMAPA | Not Heated | 100 | 55.56 | 35.71 | 30.16 | 19.84 |
| 13. | **EXP 5.** PEC. 2% MVE/MA Copolymer & PQ-55 | Heated | 100 | 82.39 | 69.72 | 66.90 | 63.38 |
| 14. | **EXP 5.** PEC, 2% MVE/MA Copolymer & PQ-55 | Not Heated | 100 | 73.28 | 51.72 | 38.79 | 17.24 |

DMAPA = dimethylaminopropyl methacrylmide

**Table 4:** Combing force reduction of PEC treated hair tresses with and without heat treatment after shampoo washing

| Polymers & PECs | Combing Force for Not Heated Hair Stress | | | Combing Force for Heated & Treated Hair Stress | | |
|---|---|---|---|---|---|---|
| | Initial | After 1 wash | Reduction After 1 wash | Initial | After 1 wash (Treated & heated) | Reduction after 1 wash (heated) |
| Untreated | 94.54 | 95.65 | -1.18% | 103.82 | 90.81 | 12.54% |
| **EXP 6:** 2% MVE/MA & PVP/ DMAPA acrylate copolymer | 103.26 | 60.19 | 41.72% | 90.98 | 54.83 | 39.74% |
| **EXP 5:** 2% MVE/MA & PQ-55 | 133.76 | 41.67 | 68.85% | 121.07 | 36.23 | 70.07% |
| 2% PVP/DMAPA acrylate copolymer | 120.20 | 44.46 | 63.01% | 116.44 | 21.17 | 81.82% |
| 2% PQ-55 | 114.67 | 35.07 | 69.41% | 113.89 | 23.86 | 79.05% |

DMAPA = dimethylaminopropyl methacrylmide

The results in Table 4 shows that PEC treated hair after washing with shampoo provides good combing force reduction, demonstrating the durable conditioning effect on the hair by the PEC composition of present application.

**EXAMPLE 14:** This example provides a styling cream formulation contains the PEC composition of Example 5 of Table 2 and the hair Salon test result of this product on a mannequin head. The left side of the head was treated with the PEC composition in Table 5, blow dried, hot flat ironed. Then the mannequin was washed with commercially available shampoo and rinsed with commercially available conditioner for 5 times and air dried between each wash and rinse. There were no repeated treatment with the PEC composition after each wash and rinse cycle.

**Table 5:** Hair Durable Styling Cream containing PEC

| Phase | Ingredients | 12460-94.13 (%) |
|---|---|---|
| A | Deionized Water | 44.8 |
| | Hydroxymethyl cellulose | 0.8 |
| | PEG-40 Hydrogenated Castor Oil | 0.7 |
| | PEG-60 hydrogenated castor oil | 0.5 |
| B | Quaternium-26 | 0.2 |
| | Propylene Glycol | 1 |
| | dimethicone | 0.5 |
| C | Phenoxyethanol (and) Caprylyl Glycol | 1.5 |
| | 12232-97.1, Example 5 | 50 |
| | Total | 100 |

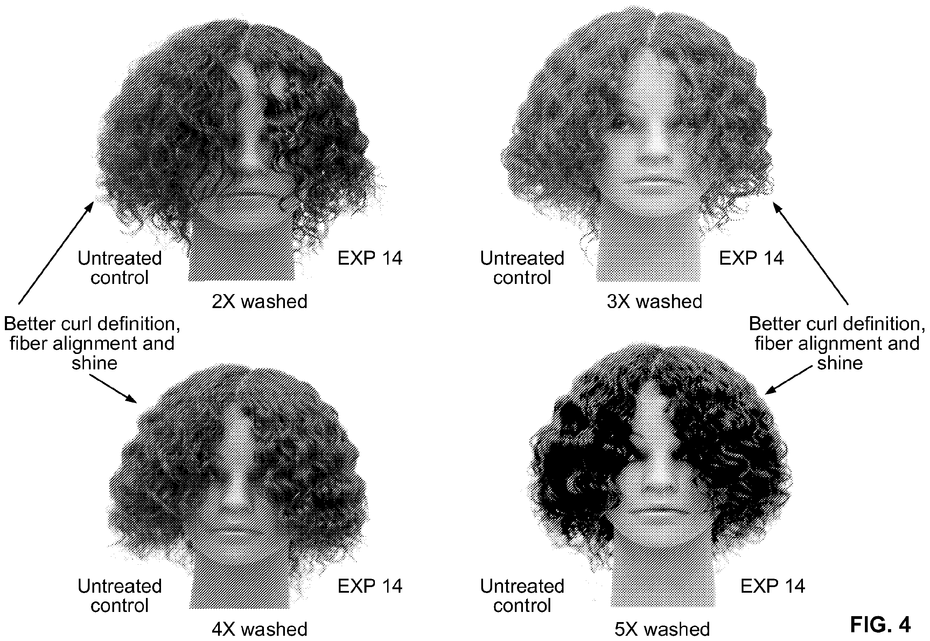

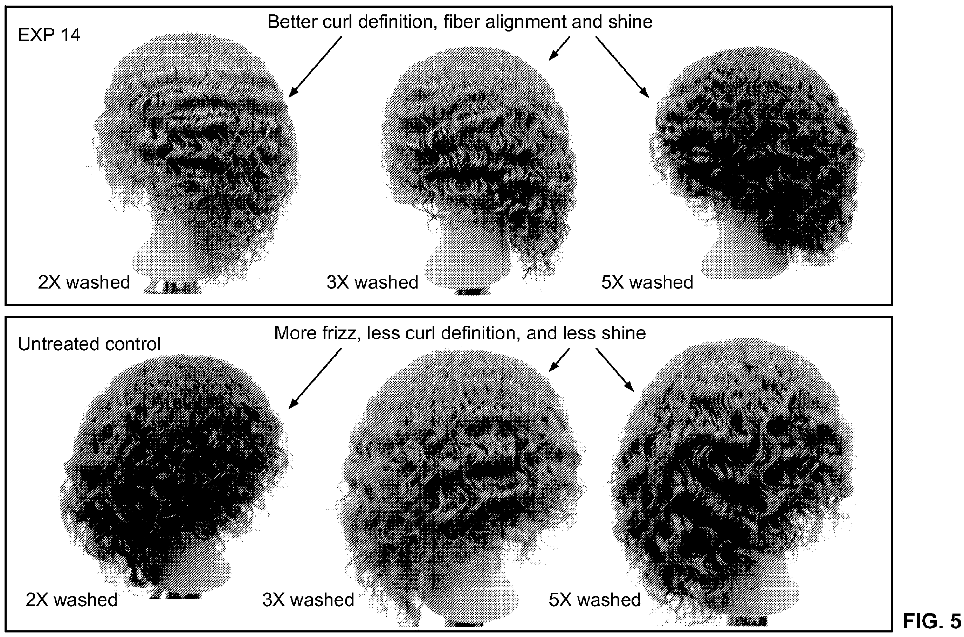

The Figures 4 and 5 show the hair salon test results with a mannequin for durable styling and wash resistant retention of hair shape benefit from a PEC durable styling cream in Table 5. These pictures demonstrate that the left side of the half head treated with PEC composition has better curl definition, fiber alignment, less frizz and better shine effect than the untreated control side (right side) which lasted for 5x wash cycles tested

**Example 15**

This example provides a styling lotion formulation contains the PEC composition of EXP 5 and its hair tress test results for durable styling benefit. The tress was treated with the PEC composition in Table 6. blow dried, hot flat ironed then washed with commercially available shampoo for 3 times and air dried between each wash.

**Table 6:** Hair Durable Styling lotion containing PEC composition

| Phase | Ingredient | 12460-94.8 |
|---|---|---|
| A | Deionized Water | 46.55 |
| | Hydroxy ethyl cellulose | 0.75 |
| B | Quaternium-26 | 0.2 |
| | Propylene Glycol | 1 |
| | Dimethicone | 0.5 |
| C | Phenoxyethanol (and) Caprylyl Glycol (and) Sorbic Acid | 1 |
| | 12232-97.1, EXP 5 | 50 |
| | Total | 100 |

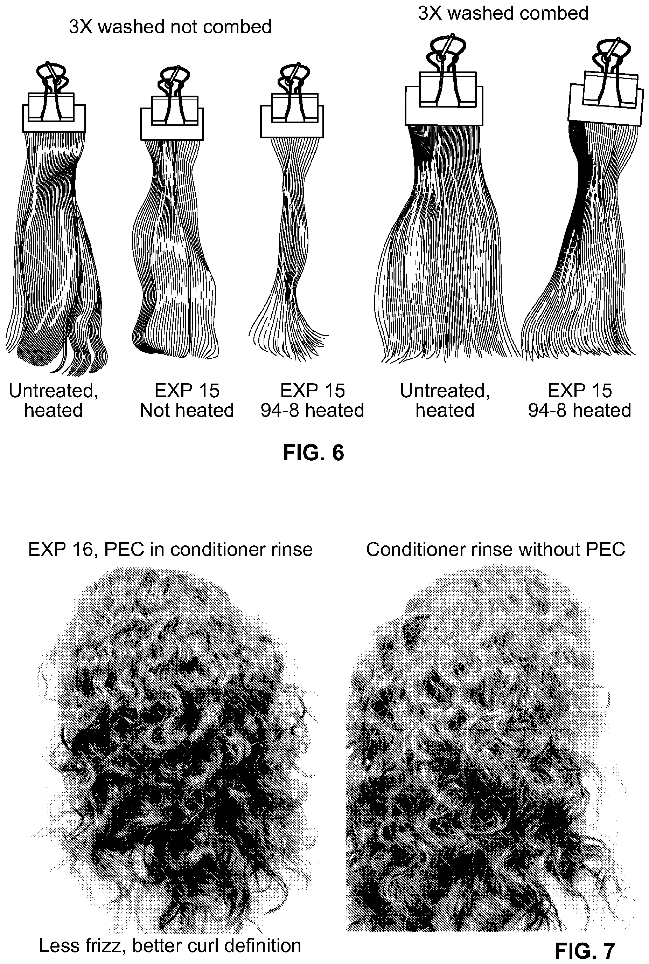

Figure 6 shows that hair tress treated with durable styling lotion containing PEC composition in Table 6 provides better fiber management, anti-fizz control, holding effect and shine effect than the untreated control after 3 wash cycles, demonstrated the durable or wash resistant styling effect of the PEC composition.

**Example 16:** This example demonstrates the consumer perceivable benefit of rinse resistant styling effect in a hair Salon test provided by the PEC compositions from a rinse off product, a conditioner rinse. The panelist hair was divided into two halves: one side is for control and the other half is for the PEC conditioner rinse formulation. The hair salon tests clearly demonstrate the rinse-resistant shape retention and styling benefit of hair fibers provided by PEC from a single conditioner rinse product in Table 7. The hair side treated from a conditioner rinse containing PEC (EXP 8) has much better curl definition, fiber alignment, less frizz and more shine as well.

**Table 7:** Formulation of a conditioner rinse containing 2% PEC

| Phase | Ingredient | % w/w |
|---|---|---|
| A | Deionized Water | 54.2 |
| | Hydroxy ethyl cellulose | 0.80 |
| | Glycerin | 1.0 |
| | Disodium EDTA | 0.1 |
| B | Cetearyl Alcohol | 3.0 |
| | Behenyl Alcohol (and) Cetearyl Alcohol (and) Hydroxyethyl Cetearamido propyldimonium Chloride | 5.0 |
| C | Concentrated PEC, EXP 8. 5.75% | 34.7 |
| D | Propylene Glycol (and) Diazolidinyl | 0.50 |
| | Total | 100 |

**Example 17:**

This example provides a durable styling cream gel formulation containing PEC for durable styling benefit.

**Table 8:** Durable styling cream gel containing PEC

| Phase | Ingredients | 12460-105.4 |
|---|---|---|
| A | Deionized Water | 55.171 |
| | Cetyl modified hydroxyethyl cellulose | 0.6 |
| | Polyquaternium-55 | 7.217 |
| | Quaternium-22 | 0.5 |
| | Propylene Glycol | 2 |
| | Butylene Glycol | 2 |
| B | Behentrimonium Methosulfate & C10-40-Isoalkylamidopropylethyl-dimonium Ethosulfate & Cetyl alcohol | 1 |
| | Glycerol stearate & PEG-100 stearate | 2 |
| | Dimethicone | 0.5 |
| C | Deionized Water | 10 |
| | Methyl vinyl ether/ maleic anhydride copolymer | 0.273 |
| | NaOH (10%) | 0.819 |
| D | Carbomer | 16.67 |
| | 2-Amino-2-methyl-1-propanol solution | 0.25 |
| E | Phenoxyethanol & Methylparaben & Isopropylparaben & Isobutylparaben & Butylparaben | 1 |
| | Total | 100 |

Method for preparing durable styling cream gel of Example 17: The below given detailed procedure is followed to prepare the said cream:

**Phase A:** Added Glycols, Quaternium -22 and PQ-55 to the main vessel with water and the contents were mixed until it becomes clear and uniform mixture. With propeller mixing slowly cetyl modified hydroxyethyl cellulose was sprinkled to completely wet it out. The Phase A was heated to 75-80°C with continuous stirring. Batch or the contents of phase were got thicken.

**Phase B:** In a separate container all Phase B ingredients were added and heated to 75-80°C with mixing. When Phase A and Phase B were at 75-80°C, the contents of Phase B were added to Phase A with vigorous mixing and for 15-20 minutes and allowed to cool to 40°C, and the mixing speed was reduced. Changing from prop to sweep mixing followed if needed. Further, it was continued to cool the contents to 35°C.

**Phase C:** The pre-made phase C ingredients were added to the main batch with mixing until it becomes uniform mixture.

**Phase D** The pre-made 1.5% of Carbomer 980 dispersion was charged to main batch and then, slowly the neutralizer was added to main batch with continuous sweep mixing.

**Phase E:** Lastly, the ingredients of phase E were added into main batch and mixing continued until it becomes uniform mixture.

**Example 18 - 20:**

This example provides a 2-in-1 shampoo formula comprising polyelectrolyte complex of EXP 12 and professional salon test results.

Six shampoos were prepared, three serving as controls, and three serving as Examples of the invention (Table 9). The polyelectrolyte complex used in the three shampoo formula was from a concentrated 8% complex (by weight of the active) of methyl vinyl ether / maleic acid copolymer and Polyquaternium-28.

Control formula I and Example 18 represent shampoos lacking cationic guar gum, which is a very commonly used conditioning agent. Control formulas 2 and 3 represent 2-in-1 shampoos in that they contain a cationic guar gum. Examples 19 and 20 resemble the 2-in-1 shampoos, but as they contain the polyelectrolyte complex, they actually are examples of 3-in-1 shampoos, in that the polyelectrolyte complex also provides auxiliary benefits.

To make the shampoos, first cocamidopropyl betaine and water were placed in to the main container equipped with a mixing and a propeller agitator. With mixing, the cationic guar gum was added and mixed until uniform with heating to 70°C. Then, the sodium laureth sulfate was blended until uniform and then the blend was cooled. The silicone was added last (where indicated).

Control 2 was tested against Example 19 in a salon on a live panelist by a professional cosmetologist having a NJ state license to practice. The results of her evaluation are found in Table 10. The test parameters were ranked from 1 (low) to 5 (high). Based on experience in evaluating products in the salon, a score difference of one (1) point between the two formulas indicates an improvement that is discernible by consumers. Hair attributes characteristic of a styling product - namely volume, body, styleability, and manageability - are higher for the shampoo having the polyelectrolyte complex. In fact, these hair attributes are significantly higher since the scores are 1 or more for the polyelectrolyte shampoo over the control. This result also is surprising, since styling polymers are expected to rinse from hair. However, the salon scores indicate clearly that the PHC has tenacity to the hair by virtue of the fact that hair styling benefits are achieved in comparison to the control formulas without the PEC.

**Table 9:** Formulas of Examples 19-20 and controls

| Ingredients | Control 1 1-n-1 | Example 18 1-in-1 | Control 2 2-in-1 | Control 3 2-in-1 w. silicone | Example 19 3-in-1 | Example 20 3-in-1 w. silicone |
|---|---|---|---|---|---|---|
| Water | q.s. | q.s. | q.s. | q.s. | q.s. | q.s. |
| Cocamidopropyl betaine, 35% | 11.43 | 11.43 | 11.43 | 11.43 | 11.43 | 11.43 |
| Cationic guar gum | - | - | 0.30 | 0.30 | 0.30 | 0.30 |
| SLES. 25% | 32.00 | 32.00 | 32.00 | 32.00 | 32.00 | 32.00 |
| Silicone/DC 1784 | - | - | - | 2.00 | - | 2.00 |
| 8% Polyelectrolyte complex | - | 50.00 | - | - | 50.00 | 50.00 |
| Total | 100.00 | 100.00 | 100.00 | 100.00 | 100.00 | 100.00 |

**Table 10:** Professional salon test results

| Hair attribute - Wet | Example 19 | Control 2 |
|---|---|---|
| Rinseability | 3.5 | 3.5 |
| Ease of wet combing | 3.5 | 3.5 |
| Smooth feel | 3.5 | 3 |

(continued)

| Hair attribute - Wet | Example 19 | Control 2 |
|---|---|---|
| Clean feel | 5 | 5 |

| Hair attribute - Dry | Example 19 | Control 2 |
|---|---|---|
| Ease of blowout | 4 | 4 |
| Ease of dry combing | 3.5* | 2.5 |
| Shine | 3 | 3 |
| Clean feel | 3 | 3 |
| Smooth feel | 3 | 3 |
| Volume | 4* | 3 |
| Body (structure) | 4* | 2 |
| Anti-frizz | 4* | 3 |
| Styleability | 4* | 3 |
| Manageability | 4* | 3 |
| Static | 1 | 1 |
| Overall appearance | 4.5* | 3 |
| Note: An asterisk (*) indicates an improvement that is discernible by consumers. | | |

**Example 21:** Mousse containing a polyelectrolyte complex

A mousse containing a polyelectrolyte complex was made following this procedure and ingredients listed in Table 11.

(1) Phase A was prepared by placing the indicated quantity of water to the main container equipped with propeller agitation. Then, the polyelectrolyte complex was added, followed by glycerine, dimethicone copolyol in that order. Each ingredient was added separately and mixed until uniform before adding the next ingredient.

(2) In a separate container the ingredients for phase B were added and mixed until uniform. Then, Phase B was added to the container with Phase A and the composition mixed until uniform.

(3) The concentrate then was charged into an aluminum-lined can and crimped closed with an appropriate mousse valve. The can was pressurized with A-46. and the actuator was fitted onto the valve.

This formula creates rich foam that spreads easily through the hair. The hair feels smooth when combed, as there is little comb drag. After styling the hair is left shiny, smooth, and will hold its shape during the course of the day. It supplies other hair shape benefits such as volume, and re-stylability.

This mousse contains 2.0% polyelectrolyte complex active. Yet, the mousse (or any hair care product) can be formulated with much less or more of the complex to help optimize the styling and care benefits from this formula. More generically, a hair care composition may comprise from about 0.10% to 8% and more particularly from about 1% to about 3%, of the polyelectrolyte complex (by weight, with respect to the active). Other ingredients can be adjusted in the formula to fine tune the properties of the hair care composition (such as lather, foam, viscosity, workability), and the hair care benefits.

**Table 11:** Mousse formula of Example 21

| Phase | Ingredients | 133-1 (%) |
|---|---|---|
| Concentrate | Water | q.s. |
| A | Polyelectrolyte complex (4% active) | 50.00 |
| | Glycerin | 1.00 |
| | Dimethicone copolyol | 0.50 |

(continued)

| Phase | Ingredients | 133-1 (%) |
|---|---|---|
| B | Polysodiate 20 | 0.50 |
| | Fragrance | 0.25 |
| | Phenoxyethanol & Caprylyl glycol | 1.00 |
| | Almond oil | 1.00 |
| Propellant | A-46 (isobutane/propane) | 6.00 |

**Example 22:** Hair spray containing a polyelectrolyte complex

A hair spray containing a polyelectrolyte complex was made having the ingredients listed in Table 12 To make the hair spray, each ingredient was added in the order listed, mixing until uniform in between each addition. The hair spray was loaded into an aluminium-lined can fitted with a Precision P1 (0.18 mL dosage, 0.012" MBU, Jumbo Dip Tube) actuator.

When the hair spray is made with the correct anionic and cationic polymers having appropriate molecular weights, charge densities, and weight ratios, a sprayable stable, and efficacious spray product results. Efficacy can be evaluated by having both enhanced care properties as well as enhanced hair shape properties due to the resinous film formed on the hair due to the polyelectrolyte complex.

**Table 12:** Hair spray formula of Example 22

| Ingredients | % wt. |
|---|---|
| Water | *q.s.* |
| 4% PEC concentrate | 25.00 |
| Disodium EDTA | 0.05 |
| Liquid Germall® Plus | 0.50 |
| Total | 100.00 |

**Claims**

1. An aqueous hair care composition capable of providing durable non-permanent hair styling and/or wash-resistant retention of shape of hair fibers comprising a polyelectrolyte complex having a gel matrix structure or a mixture of gel matrix and microgel formed by electrostatic attraction, wherein the polyelectrolyte complex comprises a complex between:

    A. 0.01 wt. % to 20 wt. % of at least one anionic polymer having a molecular weight of from 100,000 Daltons to 5000,000 Daltons and being a diacid or half ester of methyl vinyl ether / maleic anhydride (MVE/MA) copolymer; and
    B. 0.1 wt. % to 20 wt. % of at least one cationic polymer having a (i) molecular weight range of 80,000 Daltons to 5,000,000 Daltons, (ii) a charge density of less than 4.0 meg/gram and (iii) being polyquaternium-55 (PQ-55),

    wherein the charge ratio of (B) : (A) is more than 0.70.

2. The hair care composition according to claim 1, wherein said mixture of gel matrix and microgel comprises 10% of microgel to 90% of gel matrix to 90% of microgel to 10% of gel matrix, and wherein, the average particle size of gel matrix is more than 15 microns, and the average particles size of microgel is less than 7 microns.

3. The hair care composition according to claim 1, wherein

    - said gel matrix structure or a mixture of gel matrix and microgel is formed by mixing 0.01 wt. % to 20 wt. % of the anionic polymer (A) and 0.1 wt. % to 20 wt. % of the cationic polymer (B), wherein (A) is added to (B) or (B) is added to (A) at room temperature, and wherein said (A) is pre-neutralized to a pH of 5 to 10 before mixing with (B); or

- said gel matrix structure is capable of forming a water-resistant film after drying and/or after heating to a temperature of 90 °C or more; or
- the charge ratio of (B):(A) is more than 1.0.

4. The hair care composition according to claim 1, further comprising at least one cosmetically acceptable additive selected from the group consisting of: thickeners, viscosifiers, surfactants, cationic surfactants, emulsifiers, perfumes, preservatives, UV protectants, chelating agents, cleansing agents, wetting agents, hair conditioning ingredients, flexibility enhancers, split modifiers, conditioning ingredients, humectants, propellants, compressed gases, proteins, amino acids, shine enhancers, neutralizing agents, texturizing agents, water-proofing agents, solubilizers, suspension agents, suspended materials, cosmetically active ingredients, hair polymers, silicons, silicone-containing polymers, light protection agents, bleaches, gel formers, care agents, colorants, tinting agents, tanning agents, dyes, pigments, bodying agents, moisturizers, refatting agents, collagen, protein/keratin hydrolyzates, lipids, antioxidants, defoamers, antistatics, emollients, softeners, anti-dandruff agents, hair growth agents, anti-inflammatory agents, anti-microbial agents, foam stabilizers, rheology modifiers, water softening agents, hydrotropes, polyalkylene glycols, acids, bases, buffers and blends thereof, wherein

- said thickener is preferably selected from the group consisting of hydroxypropylcellulose, hydroxyethylcellulose, cetyl modified hydroxyethylcellulose, carboxymethylcellulose, methylcellulose and hydroxypropyl methylcellulose, lightly to highly crosslinked polyacrylic acid polymers, hydrophobically modified crosslinked polyacrylic acid polymers, alkali swellable crosslinked polyacrylic acid polymers, and its derivatives, and combinations thereof; or
- said cationic surfactant is preferably selected from the group consisting of quaternary ammonium compounds having at least one $C_8$ - $C_{40}$ alkyl or alkenyl group, cetyl trimethylammonium chloride, stearyl dimethyl benzyl ammonium chloride, cetylpyridinium chloride, behentrimonium methosulfate, quaternium-18, quaternium-22, quaternium-26, quaternium-31, amine quaternary ammonium compounds, dialkyoylalkyl dimethylammonium halides, primary fatty amines, secondary fatty amines, tertiary fatty amines, and combinations thereof.

5. The hair care composition according to claim 1, wherein said polyelectrolyte complex is used in an amount of from 0.1 wt. % to 10 wt. % of total composition.

6. The hair care composition according to claim 1, wherein said composition is formulated as rinse-off or leave-in type of composition.

7. The hair care composition according to claim 6, wherein said rinse-off composition is a shampoo, conditioner, hair straighteners, permanent wave or hair color, wherein

- said shampoo is preferably capable of providing ease of styling, style durability, anti-frizz, manageability, increased conditioning, better volume, and/or better appearance in comparison with a shampoo that do not comprise said polyelectrolyte complex; or
- said conditioner is preferably capable of providing greater styleability, style durability, anti-frizz, conditioning, dry conditioning, wet combing, and/or better volume in comparison with a conditioner that do not comprise said polyelectrolyte complex.

8. The hair care composition according to claim 6, wherein said leave-in composition is a mousse, a hair lacquer, a hair gel, a hair lotion, a hair wax, a styling cream, a pomade, a tonic, a hair spray, a working spray, a finishing spray, a blow-dry protectant spray, a flat-iron spray, a thermal protectant spray, or a curl-enhancing spray.

9. The hair care composition according to claim 8, wherein said leave-in type compositions are capable of providing better styleability, durability of style, anti-frizz, volume, shine, hair protection from styling appliances, better conditioning, anti-static, anti-frizz benefit in comparison with a leave-in hair care composition that does not comprise said polyelectrolyte complex.

10. A process for preparing the aqueous hair care composition according to claim 1 being capable of providing durable non-permanent hair styling and/or wash-resistant retention of shape of hair fibers, comprising the steps of:

i. preparing an aqueous solution of 0.1 wt.-% to 20 wt.-% of the at least one cationic polymer (B);
ii. adding 0.1 wt.-% to 3 wt.-% of thickeners to the solution of step (i) and mixing continuously until to form an uniform mixture;

iii. mixing the oil phase ingredients of (a) 0.1 wt. % to 10 wt. % of emulsifier, (b) 0.1 wt. % to 10 wt. % of cationic surfactants and (c) optionally, 0.5 wt. % to 5 wt. % of silicone in a different container and heating the mixture to 70 °C to 75 °C to form a uniform mixture of oil phase and adding the resultant mixture oil phase to the uniform mixture of step (ii) then combined mixture is heated at 70 °C to 75 °C with continuous mixing for 20 to 30 minutes, and allowing the resultant to cool to 35 °C and slow down mixing;

iv. neutralizing 0.01 wt.-% to 20 wt.-% of the at least one anionic polymer (A) by employing an aqueous alkali medium to bring the pH of 7.0 and adding this resultant solution to the resultant of step (iii) at 35 °C with continuous mixing until to form an uniform mixture; and

v. adding at least one 0.5 wt.-% to 5 wt.-% of preservative to the resultant of step (iv) and pass through homogenizer if required.

**11.** The process according to claim 10, wherein

- the thickener is selected from the group consisting of hydroxypropylcellulose, hydroxyethylcellulose, cetyl modified hydroxyethylcellulose, carboxymethylcellulose, methylcellulose and hydroxypropyl methylcellulose, lightly to highly crosslinked polyacrylic acid polymers, hydrophobically modified crosslinked polyacrylic acid polymers, alkali swellable crosslinked polyacrylic acid polymers, and its derivatives, and combinations thereof; or
- the cationic surfactant is selected from the group consisting of quaternary ammonium compounds having at least one $C_8$ - $C_{40}$ alkyl or alkenyl group, cetyl trimethylammonium chloride, stearyl dimethyl benzyl ammonium chloride, cetylpyridinium chloride, behentrimonium methosulfate, quaternium-18, quaternium-22, quaternium-26, quaternium-31, amine quaternary ammonium compounds, dialkyoylalkyl dimethylammonium halides, and combinations thereof; or
- the emulsifier is selected from the group consisting of condensation products of aliphatic ($C_8$ to $C_{40}$) primary or secondary linear or branched-chain alcohols or phenols with alkylene oxides, $C_6$-$C_{100}$ ethylene oxide (EO), glycol distearate, sorbitan trioleate, propylene glycol isostearate, glycol stearate, sorbitan sesquioleate, glyceryl stearate, lecithin, sorbitan oleate, sorbitan monostearate NF, sorbitan stearate, sorbitan isostearate, steareth-2, oleth-2, glyceryl laurate, ceteth-2, PEG-30 dipolyhydroxystearate, glyceryl stearate SE, sorbitan stearate, sucrose cocoate, PEG-4 dilaurate, methyl glucose sesquistearate, PEG-8 dioleate, sorbitan laurate, PEG-40 sorbitan peroleate, laureth-4, PEG-7 glyceryl cocoate, PEG-20 almond glycerides, PEG-25 hydrogenated castor oil, stearamide MEA, glyceryl stearate, PEG-100 stearate, polysorbate 85, PEG-7 olivate, cetearyl glucoside, PEG-8 oleate, polyglyceryl-3 methyglucose distearate, Oleth-10, Oleth-10 / polyoxyl 10 oleyl ether NF, ceteth-10, PEG-8 laurate, cocamide MEA, polysorbate 60 NF, polysorbate 60, polysorbate 80, isosteareth-20, PEG-60 almond glycerides, polysorbate 80 NF, PEG-20 methyl glucose sesquistearate, ceteareth-20, oleth-20, steareth-20, steareth-21, ceteth-20, isoceteth-20, polysorbate 20, polysorbate 20 NF, laureth-23, PEG-100 stearate, steareth-100, PEG-80 sorbitan laurate alone or in combinations thereof; or
- said preservative is selected from the group consisting of triazoles, imidazoles, naphthalene derivatives, benzimidazoles, morphline derivatives, dithiocarbamates, benzisothiazoles, benzamides, boron compounds, formaldehyde donors, isothiazolones, thiocyanates, quaternary ammonium compounds, iodine derivates, phenol derivatives, phenoxyethanol and caprylyl glycol, micobicides, pyridines, dialkylthiocarbamates, nitriles, parabens, alkyl parabens and salts thereof alone or in combination.

**Patentansprüche**

**1.** Eine wässrige Haarpflegezusammensetzung, die in der Lage ist, langlebiges, nicht dauerhaftes Hairstyling und/oder waschbeständige Formbeständigkeit von Haarfasern zur Verfügung zu stellen, umfassend einen Polyelektrolyt-Komplex mit einer Gel-Matrix-Struktur oder einer Mischung aus Gel-Matrix und Mikrogel, der durch elektrostatische Anziehung gebildet wird, wobei der Polyelektrolyt-Komplex einen Komplex zwischen

A. 0,01 Gew.-% bis 20 Gew.-% mindestens eines anionischen Polymers mit einem Molekulargewicht von 100 000 Dalton bis 5 000 000 Dalton, der eine Disäure oder ein Halbester von Methylvinylether/ Maleinsäure-Anhydrid (MVE/MA)-Copolymer ist; und

B. 0,1 Gew.-% bis 20 Gew.-% mindestens eines kationischen Polymers mit einem (i) Molekulargewicht im Bereich von 80 000 Dalton bis 5 000 000 Dalton und (ii) einer Ladungsdichte von weniger als 4,0 meg/Gramm, das (iii) Polyquaternium-55 (PQ-55) ist,

umfasst,

wobei das Ladungsverhältnis von (B):(A) mehr als 0,70 beträgt.

**2.** Die Haarpflegezusammensetzung gemäß Anspruch 1, wobei die Mischung aus Gel-Matrix und Mikrogel 10% des Mikrogels zu 90% der Gel-Matrix bis 90% des Mikrogels zu 10% der Gel-Matrix umfasst, und wobei die durchschnittliche Partikelgröße der Gel-Matrix mehr als 15 Mikron beträgt und die durchschnittliche Partikelgröße des Mikrogels weniger als 7 Mikron beträgt.

**3.** Die Haarpflegezusammensetzung gemäß Anspruch 1, wobei

- die Gel-Matrix-Struktur oder eine Mischung aus Gel-Matrix und Mikrogel durch Mischen von 0,01 Gew.-% bis 20 Gew.-% des anionischen Polymers (A) und 0,1 Gew.-% bis 20 Gew.-% des kationischen Polymers (B) gebildet wird, wobei (A) zu (B) oder (B) zu (A) bei Raumtemperatur hinzugefügt wird, und wobei (A) auf einen pH-Wert von 5 bis 10 vor dem Mischen mit (B) vorneutralisiert wird; oder
- die Gel-Matrix-Struktur in der Lage ist, nach dem Trocknen und/oder nach dem Erhitzen auf eine Temperatur von 90 °C oder mehr einen wasserbeständigen Film zu bilden; oder
- das Ladungsverhältnis von (B):(A) mehr als 1,0 beträgt.

**4.** Die Haarpflegezusammensetzung gemäß Anspruch 1, die weiterhin ein kosmetisch verträgliches Additiv ausgewählt aus der Gruppe bestehend aus: Verdickungsmitteln, Viskosifizierern, Tensiden, kationischen Tensiden, Emulgatoren, Duftstoffen, Konservierungsmitteln, UV-Schutzmitteln, Komplexbildnern, Reinigungsmitteln, Benetzungsmitteln, Haarkonditionierungsinhaltsstoffen, Flexibilitätsverstärkern, Split-Modifikatoren, Konditionierungsinhaltsstoffen, Feuchthaltemitteln, Treibmitteln, Druckgasen, Proteinen, Aminosäuren, Glanzverstärkern, Neutralisationsmitteln, Texturierungsmitteln, Hydrophobierungsmitteln, Lösungsmittelvermittlern, Suspensionsmitteln, Schwebstoffen, kosmetischen Wirkstoffen, Haarpolymeren, Silikonen, silikonhaltigen Polymeren, Lichtschutzmitteln, Bleichmitteln, Gelbildnern, Pflegemitteln, Farbstoffen, Tönungsmitteln, Gerbstoffen, Farbstoffen, Pigmenten, Körperpflegemitteln, Feuchtigkeitsspendern, Rückfettungsmitteln, Kollagen, Protein-/Keratin-Hydrolysaten, Lipiden, Antioxidationsmitteln, Entschäumern, Antistatika, Emollienzien, Weichmachern, Antischuppenmitteln, Haarwuchsmitteln, entzündungshemmenden Mitteln, antimikrobiellen Mitteln, Schaumstabilisatoren, Rheologie-Modifikatoren, Wasserenthärtern, Hydrotropen, Polyalkylenglykolen, Säuren, Basen, Puffern und Mischungen davon, wobei

- das Verdickungsmittel bevorzugt ausgewählt wird aus der Gruppe bestehend aus Hydroxypropylcellulose, Hydroxyethylcellulose, Cetyl-modifizierter Hydroxyethylcellulose, Carboxymethylcellulose, Methylcellulose und Hydroxypropylmethylcellulose, leicht bis stark vernetzten Polyacrylsäurepolymeren, hydrophob modifizierten vernetzten Polyacrylsäurepolymeren, alkalisch quellbaren vernetzten Polyacrylsäurepolymeren und deren Derivate und deren Kombinationen; oder
- das kationische Tensid bevorzugt ausgewählt wird aus der Gruppe bestehend aus quaternären Ammoniumverbindungen mit mindestens einem $C_8$ - $C_{40}$-Alkyl- oder - Alkenylrest, Cetyltrimethylammoniumchlorid, Stearyldimethylbenzylammoniumchlorid, Cetylpyridiniumchlorid, Behentrimoniummethosulfat, Quaternium-18, Quaternium-22, Quaternium-26, Quaternium-31, Amin-quaternären Ammoniumverbindungen, Dialkyoylalkyldimethylammoniumhalogeniden, primären Fettaminen, sekundären Fettaminen, tertiären Fettaminen und deren Kombinationen.

**5.** Die Haarpflegezusammensetzung gemäß Anspruch 1, wobei der Polyelektrolyt-Komplex in einer Menge von 0,1 Gew.-% bis 10 Gew.-% der Gesamtzusammensetzung verwendet wird.

**6.** Die Haarpflegezusammensetzung gemäß Anspruch 1, wobei die Zusammensetzung als Rinse-off- oder Leave-in-Zusammensetzung formuliert wird.

**7.** Die Haarpflegezusammensetzung gemäß Anspruch 6, wobei die Rinse-off-Zusammensetzung ein Shampoo, eine Spülung, ein Haarglättmittel, eine Dauerwelle oder eine Haarfarbe ist, wobei

- das Shampoo bevorzugt in der Lage ist, einfaches Frisieren, Haltbarkeit der Frisur, Anti-Frizz, Handhabbarkeit, erhöhte Konditionierung, besseres Volumen und/oder besseres Aussehen im Vergleich zu einem Shampoo, das den Polyelektrolyt-Komplex nicht umfasst, zu bieten; oder
- die Spülung bevorzugt in der Lage ist, eine größere Frisierbarkeit, Haltbarkeit der Frisur, Anti-Frizz, Konditionierung, Trockenkonditionierung, Nasskämmbarkeit und/oder besseres Volumen im Vergleich zu einer Spülung, die den Polyelektrolyt-Komplex nicht umfasst, zu bieten.

**8.** Die Haarpflegezusammensetzung gemäß Anspruch 6, wobei die Leave-in-Zusammensetzung ein Mousse, ein Haarlack, ein Haargel, eine Haarlotion, ein Haarwachs, eine Styling-Creme, eine Pomade, ein Tonikum, ein Haarspray, ein Working-Spray, ein Finishing-Spray, ein Föhn-Schutzmittel-Spray, ein Glätteisen-Spray, ein Wärmeschutz-Spray oder ein Lockenstab-Spray ist.

9. Die Haarpflegezusammensetzung gemäß Anspruch 8, wobei die Leave-in-Zusammensetzungen in der Lage sind, eine bessere Frisierbarkeit, Haltbarkeit der Frisur, Anti-Frizz, Volumen, Glanz, Haarschutz vor Styling-Geräten, bessere Konditionierung, antistatische, Anti-Frizz-Vorteile im Vergleich zu einer Leave-in-Haarpflegezusammensetzung, die den Polyelektrolyt-Komplex nicht umfasst, zu bieten.

10. Ein Verfahren zur Herstellung einer wässrigen Haarpflegezusammensetzung gemäß Anspruch 1, die in der Lage ist, haltbares, nicht dauerhaftes Hairstyling und/oder waschbeständige Formbeständigkeit von Haarfasern zur Verfügung zu stellen, umfassend die Schritte:

> i. Herstellen einer wässrigen Lösung von 0,1 Gew.-% bis 20 Gew.-% wenigstens eines kationischen Polymers (B);
> ii. Zugabe von 0,1 Gew.-% bis 3 Gew.-% Verdickungsmittel zu der Lösung aus Schritt (i) und kontinuierliches Mischen, bis eine gleichförmige Mischung gebildet wird;
> iii. Mischen der Bestandteile der Ölphase aus (a) 0,1 Gew.-% bis 10 Gew.-% Emulgator, (b) 0,1 Gew.-% bis 10 Gew.-% kationische Tenside und (c) gegebenenfalls 0,5 Gew.-% bis 5 Gew.-% Silikon in einem anderen Behälter und Erhitzen der Mischung auf 70 °C bis 75 °C, um eine gleichmäßige Mischung der Ölphase zu bilden, und Zugabe der erhaltenen gemischten Ölphase zu der gleichförmigen Mischung aus Schritt (ii), Erhitzen der kombinierten Mischung auf 70 °C bis 75 °C unter kontinuierlichem Mischen für 20 bis 30 Minuten und Abkühlen des resultierenden Produkts auf 35 °C und Verlangsamen des Mischens;
> iv. Neutralisieren von 0,01 Gew.-% bis 20 Gew.-% des mindestens einen anionischen Polymers (A) durch Verwenden eines wässrigen alkalischen Mediums, um den pH-Wert auf 7,0 einzustellen, und Hinzufügen der erhaltenen Lösung zu dem erhaltenen Produkt aus Schritt (iii) bei 35 °C unter kontinuierlichem Mischen, bis eine gleichförmige Mischung gebildet wird; und
> v. Zugabe von mindestens 0,5 Gew.-% bis 5 Gew.-% Konservierungsmittel zu dem resultierenden Produkt aus Schritt (iv) und, sofern nötig, Leiten durch einen Homogenisator.

11. Das Verfahren gemäß Anspruch 10, wobei

> - das Verdickungsmittel ausgewählt wird aus der Gruppe bestehend aus Hydroxypropylcellulose, Hydroxyethylcellulose, Cetyl-modifizierter Hydroxyethylcellulose, Carboxymethylcellulose, Methylcellulose und Hydroxypropylmethylcellulose, leicht bis stark vernetzten Polyacrylsäurepolymeren, hydrophob modifizierten vernetzten Polyacrylsäurepolymeren, alkalisch quellbaren vernetzten Polyacrylsäurepolymeren und deren Derivate und deren Kombinationen; oder
> - das kationische Tensid ausgewählt wird aus der Gruppe bestehend aus quaternären Ammoniumverbindungen mit mindestens einem $C_8$ - $C_{40}$-Alkyl- oder -Alkenylrest, Cetyltrimethylammoniumchlorid, Stearyldimethylbenzylammoniumchlorid, Cetylpyridiniumchlorid, Behentrimoniummethosulfat, Quaternium-18, Quaternium-22, Quaternium-26, Quaternium-31, Amin-quaternären Ammoniumverbindungen, Dialkyoylalkyldimethylammoniumhalogeniden und deren Kombinationen; oder
> - der Emulgator ausgewählt wird aus der Gruppe bestehend aus Kondensationsprodukten von aliphatischen ($C_8$ bis $C_{40}$) primären oder sekundären, linearen oder verzweigten Alkoholen oder Phenolen mit Alkylenoxiden, $C_6$-$C_{100}$ Ethylenoxid (EO), Glykoldistearat, Sorbitantrioleat, Propylenglykolisostearat, Glykolstearat, Sorbitansesquioleat, Glycerinstearat, Lecithin, Sorbitanoleat, Sorbitanmonostearat NF, Sorbitanstearat, Sorbitisostearat, Steareth-2, Oleth-2, Glyceryllaurat, Ceteth-2, PEG-30 Dipolyhydroxystearat, Glycerinstearat SE, Sorbitanstearat, Sucrosecocoat, PEG-4 Dilaurat, Methylglucosesesquistearat, PEG-8 Dioleat, Sorbitanlaurat, PEG-40 Sorbitanperoleat, Laureth-4, PEG-7 Glycerylcocoat, PEG-20 Mandelglyceride, PEG-25 hydriertes Rizinusöl, Stearamid MEA, Glycerylstearat, PEG-100 Stearat, Polysorbat 85, PEG-7 Olivat, Cetearylglucosid, PEG-8 Oleat, Polyglyceryl-3-methylglucosedistearat, Oleth-10, Oleth-10 / Polyoxyl 10 Oleylether NF, Ceteth-10, PEG-8 Laurat, Cocamid MEA, Polysorbat 60 NF, Polysorbat 60, Polysorbat 80, Isosteareth-20, PEG-60 Mandelglyceride, Polysorbat 80 NF, PEG-20 Methylglucosesesquistearat, Ceteareth-20, Oleth-20, Steareth-20, Steareth-21, Ceteth-20, Isoceteth-20, Polysorbat 20, Polysorbat 20 NF, Laureth-23, PEG-100 Stearat, Steareth-100, PEG-80 Sorbitanlaurat allein oder in deren Kombinationen; oder
> - das Konservierungsmittel ausgewählt wird aus der Gruppe bestehend aus Triazolen, Imidazolen, Naphthalenderivaten, Benzimidazolen, Morpholinderivativen, Dithiocarbamaten, Benzisothiazolen, Benzamiden, Borverbindungen, Formaldehyd-Donatoren, Isothiazolonen, Thiocyanaten, quaternären Ammoniumverbindungen, Iod-Derivate, Phenol-Derivaten, Phenoxyethanol und Caprylylglykol, Mikrobioziden, Pyridinen, Dialkylthiocarbamaten, Nitrilen, Parabenen, Alkylparabenen und deren Salze allein oder in Kombination.

**Revendications**

1. Composition aqueuse de soin des cheveux capable de fournir un coiffage des cheveux non permanent durable et/ou une rétention, résistante au lavage, de la forme des fibres capillaires, comprenant un complexe de polyélectrolytes ayant une structure de matrice de gel ou un mélange d'une matrice de gel et d'un micro-gel formé(e) par attraction électrostatique, où le complexe de polyélectrolytes comprend un complexe entre :

   A. de 0,01% en poids à 20% en poids d'au moins un polymère anionique ayant un poids moléculaire allant de 100 000 Daltons à 5 000 000 Daltons et étant un diacide ou un demi-ester d'un copolymère de méthylvinyl-éther/anhydride maléique (MVE/MA) ; et
   B. de 0,1% en poids à 20% en poids d'au moins un polymère cationique ayant (i) une plage de poids moléculaire allant de 80 000 Daltons à 5 000 000 Daltons, (ii) une densité de charge inférieure à méq/gramme et (iii) qui est un polyquaternium-55 (PQ-55) ;

   où le rapport de charge de (B):(A) est supérieur à 0,70.

2. Composition de soin des cheveux selon la revendication 1, dans laquelle ledit mélange d'une matrice de gel et d'un micro-gel comprend de 10% de micro-gel à 90% de matrice de gel à de 90% de micro-gel à 10% de matrice de gel, et où la taille de particule moyenne de la matrice de gel est supérieure à 15 microns, et la taille de particule moyenne du micro-gel est inférieure à 7 microns.

3. Composition de soin des cheveux selon la revendication 1, dans laquelle

   - ladite structure de matrice de gel ou un mélange d'une matrice de gel et d'un micro-gel est formé(e) par le mélange de 0,01% en poids à 20% en poids du polymère anionique (A) et de 0,1% en poids à 20% en poids du polymère cationique (B), où (A) est ajouté à (B) ou (B) est ajouté à (A) à température ambiante, et où ledit (A) est pré-neutralisé jusqu'à un pH allant de 5 à 10 avant le mélange avec (B) ; ou
   - ladite structure de matrice de gel est capable de former un film résistant à l'eau après séchage et/ou après chauffage jusqu'à une température de 90°C ou plus ; ou
   - le rapport de charge de (B):(A) est supérieur à 1,0.

4. Composition de soin des cheveux selon la revendication 1, comprenant en outre au moins un additif acceptable sur le plan cosmétique, choisi dans le groupe constitué par : les épaississants, les améliorants de viscosité, les agents tensioactifs, les agents tensioactifs cationiques, les émulsifiants, les parfums, les conservateurs, les agents anti-UV, les agents chélatants, les agents nettoyants, les agents mouillants, les ingrédients de conditionnement des cheveux, les améliorateurs de flexibilité, les agents de modification des fourches, les ingrédients de conditionnement, les humectants, les propulseurs, les gaz comprimés, les protéines, les acides aminés, les améliorateurs de brillant, les agents neutralisants, les agents de texture, les agents hydrofuges, les agents solubilisants, les agents de suspension, les matériaux en suspension, les ingrédients actifs sur le plan cosmétique, les polymères capillaires, les silicones, les polymères siliconés, les agents de photoprotection, les agents de blanchiment, les agents formateurs de gel, les agents de soin, les colorants, les agents de teinture, les agents de tannage, les teintures, les pigments, les agents donnant du corps, les agents hydratants, les agents relipidants, le collagène, les hydrolysats de protéine/kératine, les lipides, les antioxydants, les agents antimousse, les agents antistatiques, les émollients, les adoucissants, les agents antipelliculaires, les agents de croissance capillaire, les agents anti-inflammatoires, les agents antimicrobiens, les stabilisateurs de mousse, les modificateurs de rhéologie, les agents d'adoucissement de l'eau, les hydrotropes, les polyalkylène glycols, les acides, les bases, les tampons et des mélanges de ceux-ci, où

   - ledit épaississant est choisi de préférence dans le groupe constitué par l'hydroxypropyl cellulose, l'hydroxyéthyl cellulose, l'hydroxyéthyl cellulose à modification cétyle, la carboxyméthyl cellulose, la méthylcellulose et l'hydroxypropyl méthylcellulose, les polymères d'acide polyacrylique légèrement à hautement réticulés, les polymères d'acide polyacrylique réticulés et modifiés de manière hydrophobe, les polymères d'acide polyacrylique réticulés et gonflables aux alcalis, et leurs dérivés, et des combinaisons de ceux-ci ; ou
   - ledit agent tensioactif cationique est choisi de préférence dans le groupe constitué par les composés d'ammonium quaternaire ayant au moins un groupement $C_8$-$C_{40}$-alkyle ou alcényle, le chlorure de cétyltriméthylammonium, le chlorure de stéaryldiméthylbenzylammonium, le chlorure de cétylpyridinium, le méthosulfate de béhentrimonium, le quaternium-18, le quaternium-22, le quaternium-26, le quaternium-31, les composés d'ammonium quaternaire et d'amines, les halogénures de dialkyoylalkyl diméthylammonium, les amines grasses primaires, les amines grasses secondaires, les amines grasses tertiaires, et des combinaisons de ceux-ci.

**5.** Composition de soin des cheveux selon la revendication 1, dans laquelle ledit complexe de polyélectrolytes est utilisé selon une quantité allant de 0,1% en poids à 10% en poids de la composition totale.

**6.** Composition de soin des cheveux selon la revendication 1, où ladite composition est formulée sous forme d'une composition de type à rincer ou sans rinçage.

**7.** Composition de soin des cheveux selon la revendication 6, où ladite composition à rincer est un shampooing, un après-shampooing, des produits de lissage des cheveux, une permanente ou une teinture pour cheveux, où

- ledit shampooing est capable, de préférence, d'apporter une facilité de coiffage, une durabilité de coiffure, un effet anti-frisottis, une maniabilité, un conditionnement accru, un meilleur volume, et/ou un meilleur aspect par rapport à un shampooing qui ne comprend pas ledit complexe de polyélectrolytes ; ou
- ledit après-shampooing est capable, de préférence, d'apporter une plus grande facilité de coiffage, une durabilité de coiffure, un effet anti-frisottis, un conditionnement, un conditionnement à sec, un peignage sur cheveux humides, et/ou un meilleur volume par rapport à un après-shampooing qui ne comprend pas ledit complexe de polyélectrolytes.

**8.** Composition de soin des cheveux selon la revendication 6, où ladite composition sans rinçage est une mousse, une laque, un gel capillaire, une lotion capillaire, une cire capillaire, une crème de coiffage, une pommade, un tonique, un spray pour cheveux, un spray de travail, un spray de finition, un spray de protection pour brushings, un spray de protection pour fers à lisser, un spray de protection thermique, un spray d'amélioration des boucles.

**9.** Composition de soin des cheveux selon la revendication 8, où lesdites compositions de type sans rinçage sont capables de fournir de meilleur(e)s aptitude au coiffage, durabilité de coiffure, effet anti-frisottis, volume, brillant, protection des cheveux vis-à-vis des appareils de coiffage, un meilleur conditionnement, un bénéfice antistatique, anti-frisottis, par rapport à une composition de soin des cheveux sans rinçage qui ne comprend pas ledit complexe de polyélectrolytes.

**10.** Procédé de préparation de la composition aqueuse de soin des cheveux selon la revendication 1, étant capable de fournir un coiffage des cheveux non permanent durable et/ou une rétention, résistante au lavage, de la forme des fibres capillaires, comprenant les étapes consistant à :

i. préparer une solution aqueuse de 0,1% en poids à 20% en poids du au moins un polymère cationique (B) ;
ii. ajouter de 0,1% en poids à 3% en poids d'épaississants à la solution de l'étape (i) et mélanger de manière continue jusqu'à la formation d'un mélange uniforme ;
iii. mélanger les ingrédients de phase huileuse de (a) 0,1% en poids à 10% en poids d'émulsifiant, (b) de 0,1% en poids à 10% en poids d'agents tensioactifs cationiques et (c) éventuellement, de 0,5% en poids à 5% en poids de silicone dans un conteneur différent et chauffer le mélange jusqu'à de 70°C à 75°C afin de former un mélange uniforme de phase huileuse et ajouter la phase huileuse du mélange résultant au mélange uniforme de l'étape (ii) puis le mélange combiné est chauffé jusqu'à de 70°C à 75°C sous agitation continue pendant de 20 à 30 minutes, et on laisse le mélange résultant refroidir jusqu'à 35°C et on ralentit l'agitation ;
iv. neutraliser de 0,01% en poids à 20% en poids du au moins un polymère anionique (A) en utilisant un milieu alcalin aqueux afin d'amener le pH jusqu'à 7,0 et ajouter cette solution résultante au mélange résultant de l'étape (iii) à 35°C sous agitation continue jusqu'à la formation d'un mélange uniforme ; et
v. ajouter au moins un 0,5% en poids à 5% en poids de conservateur au mélange résultant de l'étape (iv) et faire passer dans un homogénéisateur si nécessaire.

**11.** Procédé selon la revendication 10, dans lequel

- l'épaississant est choisi dans le groupe constitué par l'hydroxypropyl cellulose, l'hydroxyéthyl cellulose, l'hydroxyéthyl cellulose à modification cétyle, la carboxyméthyl cellulose, la méthylcellulose et l'hydroxypropyl méthylcellulose, les polymères d'acide polyacrylique légèrement à hautement réticulés, les polymères d'acide polyacrylique réticulés et modifiés de manière hydrophobe, les polymères d'acide polyacrylique réticulés et gonflables aux alcalis, et leurs dérivés, et des combinaisons de ceux-ci ; ou

- l'agent tensioactif cationique est choisi dans le groupe constitué par les composés d'ammonium quaternaire ayant au moins un groupement $C_8$-$C_{40}$-alkyle ou alcényle, le chlorure de cétyltriméthylammonium, le chlorure de stéaryldiméthylbenzylammonium, le chlorure de cétylpyridinium, le méthosulfate de béhentrimonium, le quaternium-18, le quaternium-22, le quaternium-26, le quaternium-31, les composés d'ammonium quaternaire et d'amines, les halogénures de dialkyoylalkyl diméthylammonium, et des combinaisons de ceux-ci ; ou

- l'émulsifiant est choisi dans le groupe constitué par les produits de condensation de phénols ou d'alcools à chaîne linéaire ou ramifiée primaires ou secondaires en ($C_8$ à $C_{40}$) aliphatiques avec des oxydes d'alkylène, l'oxyde d'éthylène en $C_6$-$C_{100}$ (OE), le distéarate de glycol, le trioléate de sorbitane, l'isostéarate de propylène glycol, le stéarate de glycol, le sesquioléate de sorbitane, le stéarate de glycéryle, la lécithine, l'oléate de sorbitane, le monostéarate de sorbitane NF, le stéarate de sorbitane, l'isostéarate de sorbitane, le stéareth-2, l'oleth-2, le laurate de glycéryle, le céteth-2, le dipolyhydroxystéarate de PEG-30, le stéarate de glycéryle SE, le stéarate de sorbitane, le cocoate de saccharose, le dilaurate de PEG-4, le sesquistéarate de méthyl glucose, le dioléate de PEG-8, le laurate de sorbitane, le per-oléate de sorbitane et de PEG-40, le laureth-4, le cocoate de glycéryle et de PEG-7, les glycérides d'amande PEG-20, l'huile de ricin hydrogénée PEG-25, le stéaramide MEA, le stéarate de glycéryle, le stéarate de PEG-100, le polysorbate 85, l'olivate de PEG-7, le glucoside de cétéaryle, l'oléate de PEG-8, le distéarate de méthyl glucose et de polyglycéryl-3, l'oleth-10, l'oleth-10/ polyoxyl 10 oléyl éther NF, le céteth-10, le laurate de PEG-8, le cocamide MEA, le polysorbate 60 NF, le polysorbate 60, le polysorbate 80, l'isostéareth-20, les glycérides d'amande PEG-60, le polysorbate 80 NF, le sesquistéarate de méthyl glucose et de PEG-20, le cétéareth-20, l'oleth-20, le stéareth-20, le stéareth-21, le céteth-20, l'isocéteth-20, le polysorbate 20, le polysorbate 20 NF, le laureth-23, le stéarate de PEG-100, le stéareth-100, le laurate de sorbitan et de PEG-80, seuls ou selon des combinaisons de ceux-ci ; ou

- ledit conservateur est choisi dans le groupe constitué par les triazoles, les imidazoles, les dérivés de naphtalène, les benzimidazoles, les dérivés de morpholine, les dithiocarbamates, les benzisothiazoles, les benzamides, les composés de bore, les donneurs de formaldéhyde, les isothiazolones, les thiocyanates, les composés d'ammonium quaternaire, les dérivés d'iode, les dérivés de phénol, le phénoxyéthanol et le caprylyl glycol, les microbicides, les pyridines, les dialkylthiocarbamates, les nitriles, les parabènes, les alkyl parabènes et les sels de ceux-ci, seuls ou selon des combinaisons.

**FIG. 1**

**FIG. 2**

FIG. 3

FIG. 4

FIG. 5

2X washed

3X washed

5X washed

Untreated control

More frizz, less curl definition, and less shine

2X washed

3X washed

5X washed

EXP 14

Better curl definition, fiber alignment and shine

3X washed not combed

3X washed combed

| Untreated, heated | EXP 15 Not heated | EXP 15 94-8 heated | Untreated, heated | EXP 15 94-8 heated |

**FIG. 6**

EXP 16, PEC in conditioner rinse

Conditioner rinse without PEC

Less frizz, better curl definition

**FIG. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20111269778 A1
- US 7837983 B2
- US 20120213723 A
- WO 2012075274 A1
- US 20060188468 A1
- WO 2013064596 A1
- US 5073614 A
- US 5312619 A
- US 5139770 A
- US 5716634 A
- US 5470884 A
- US 5759524 A
- US 5997887 A
- US 6024942 A
- US 1026973 W
- US 1026976 W
- US 1026940 W
- US 1132993 W
- US 1134515 W

### Non-patent literature cited in the description

- *Official Journal of the European Union, Commission Decision,* 09 February 2006
- CTFA Cosmetic Ingredient Handbook. 41-42
- History of Polymers in Haircare. Cosmetics and Toiletries, 1988, vol. 103
- Conditioning Agents for Skin and Hair. Cosmetic Science and Technology Series. Marcel Dekker Publishers, 1999, vol. 21
- **P.D. DORGAN.** *Drug and Cosmetic Industry,* December 1983, 30-33
- Kirk-Othmer Encyclopedia of Chemical Technology. vol. 5, 857-884
- **T. E. FURIA.** CRC Handbook of Food Additives. 1972, 271-294
- Encyclopedia of Food Science. 1978, 694-699